(19) **Europäisches Patentamt / European Patent Office / Office européen des brevets**

(11) **EP 4 268 952 A1**

(12) **EUROPEAN PATENT APPLICATION**
published in accordance with Art. 153(4) EPC

(43) Date of publication:
01.11.2023 Bulletin 2023/44

(21) Application number: 21915254.3

(22) Date of filing: 24.12.2021

(51) International Patent Classification (IPC):
$B01J\ 27/132^{(2006.01)}$    $B01J\ 27/138^{(2006.01)}$
$B01J\ 37/00^{(2006.01)}$    $B01J\ 37/08^{(2006.01)}$
$C07B\ 61/00^{(2006.01)}$    $C07C\ 253/24^{(2006.01)}$
$C07C\ 253/26^{(2006.01)}$    $C07C\ 255/08^{(2006.01)}$

(52) Cooperative Patent Classification (CPC):
B01J 27/132; B01J 27/138; B01J 37/00;
B01J 37/08; C07B 61/00; C07C 253/24;
C07C 253/26; C07C 255/08; Y02P 20/52

(86) International application number:
PCT/JP2021/048409

(87) International publication number:
WO 2022/145394 (07.07.2022 Gazette 2022/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.12.2020 JP 2020218492

(71) Applicant: Asahi Kasei Kabushiki Kaisha
Tokyo 1000006 (JP)

(72) Inventors:
• KOIKE, Natsume
  Tokyo 100-0006 (JP)
• TOMODA, Atsushi
  Tokyo 100-0006 (JP)

(74) Representative: dompatent von Kreisler Selting Werner -
Partnerschaft von Patent- und Rechtsanwälten mbB
Deichmannhaus am Dom
Bahnhofsvorplatz 1
50667 Köln (DE)

(54) **CATALYST FOR VAPOR-PHASE CATALYTIC AMMOXIDATION REACTION AND METHOD FOR PRODUCING CATALYST FOR VAPOR-PHASE CATALYTIC AMMOXIDATION REACTION**

(57) A catalyst for gas-phase catalytic ammoxidation reaction, the catalyst containing a metal oxide and a silica carrier carrying the metal oxide, wherein
the metal oxide contains molybdenum, and
an interaction coefficient C between water vapor and the catalyst for gas-phase catalytic ammoxidation reaction, the interaction coefficient C being derived by subjecting the catalyst for gas-phase catalytic ammoxidation reaction to a water vapor adsorption measurement, is 1 or more and 30 or less.

EP 4 268 952 A1

**Description**

Technical Field

The present invention relates to a catalyst for gas-phase catalytic ammoxidation reaction and a method for producing a catalyst for gas-phase catalytic ammoxidation reaction.

Background Art

A known method for producing an unsaturated nitrile includes performing gas-phase catalytic ammoxidation reaction of an unsaturated hydrocarbon such as propylene or isobutene or a saturated hydrocarbon such as propane or isobutane as a starting material. A method for improving reaction results with respect to such a starting material is variously studied, and as the latter example, proposed is to use dry silica (powder silica) to result in an increase in fluidity of a catalyst and improvements in conversion of propane and rate of selection of acrylonitrile in fluidized bed reaction (see, for example, Patent Literature 1).

Citation List

Patent Literature

[Patent Literature 1] Japanese Patent Laid-Open No. 2002-219362

Summary of Invention

Technical Problem

Patent Literature 1 discloses the pore volume and the average primary particle diameter of a catalyst carrier; however, it does not disclose sufficient studies on physical properties of the surface of the catalyst carrier. That is, the conventional art still has room for improvement in view of optimization of physical properties of a catalyst surface serving as a reaction field and further increases in reaction results.
While a technique for allowing a composite metal oxide serving as a catalyst component to be carried on a silica carrier is known as in Patent Literature 1 in view of an increase in strength of a catalyst particle and adjustment of the density thereof, it has been determined according to studies by the present inventors that there is a tendency that a silica carrier is introduced to thereby cause an increase in hydrophilicity of the entire catalyst particle and inhibition of reaction due to water vapor. Such a tendency is considered to be due to an increase in hydrophilicity by a silanol group on a silica carrier surface.
The present invention has been made in consideration of the problems, and an object of the present invention is to provide a catalyst for gas-phase catalytic ammoxidation reaction and a method for producing a catalyst for gas-phase catalytic ammoxidation reaction, in which ammonia and hydrocarbon as starting materials can efficiently be utilized and the yield can also be improved in the gas-phase catalytic ammoxidation reaction of a saturated hydrocarbon or an unsaturated hydrocarbon.

Solution to Problem

The present inventors have conducted diligent studies in order to solve the problems to find that the problems can be solved by controlling hydrophobicity of a catalyst surface with a predetermined parameter as an index, in a catalyst for gas-phase catalytic ammoxidation reaction, including a silica carrier, thereby completed the present invention.
That is, the present invention includes the following aspects.

[1] A catalyst for gas-phase catalytic ammoxidation reaction, the catalyst comprising a metal oxide and a silica carrier carrying the metal oxide, wherein

the metal oxide comprises molybdenum, and
an interaction coefficient C between water vapor and the catalyst for gas-phase catalytic ammoxidation reaction, the interaction coefficient C being derived by subjecting the catalyst for gas-phase catalytic ammoxidation reaction to a water vapor adsorption measurement, is 1 or more and 30 or less.

[2] The catalyst for gas-phase catalytic ammoxidation reaction according to [1], wherein a mass proportion of the silica carrier is 30% by mass or more and 70% by mass or less in terms of $SiO_2$ based on a total amount of the catalyst for gas-phase catalytic ammoxidation reaction.

[3] The catalyst for gas-phase catalytic ammoxidation reaction according to [1] or [2], wherein the metal oxide is represented by the following formula (1):

$$MO_1V_aSb_bNb_cW_dX_eO_n ... \qquad (1)$$

wherein X represents at least one selected from the group consisting of Te, Ce, Ti and Ta; a, b, c, and d satisfy relational expressions of $0.01 \leq a \leq 1$, $0.01 \leq b \leq 1$, $0.01 \leq c \leq 1$, $0 \leq d \leq 1$, and $0 \leq e \leq 1$; and n represents a number determined by valences of the other elements.

[4] The catalyst for gas-phase catalytic ammoxidation reaction according to [1] or [2], wherein the metal oxide is represented by the following formula (2):

$$Mo_{12}Bi_fFe_gX_hY_iZ_jO_m ... \qquad (2)$$

wherein X represents at least one element selected from the group consisting of nickel, cobalt, magnesium, calcium, zinc, strontium and barium; Y represents at least one element selected from the group consisting of cerium, chromium, lanthanum, neodymium, yttrium, praseodymium, samarium, aluminum, gallium and indium; Z represents at least one element selected from the group consisting of potassium, rubidium and cesium; f, g, h, i, j and m represent atomic ratios of the elements, and respectively satisfy $0.1 \leq f \leq 2.0$, $0.1 \leq g \leq 3.0$, $0.1 \leq h \leq 10.0$, $0.1 \leq i \leq 3.0$, and $0.01 \leq j \leq 2.0$; and m represents a number of oxygen atoms necessary for satisfying valence requirements of the other elements present.

[5] The catalyst for gas-phase catalytic ammoxidation reaction according to any one of [1] to [4], further comprising fluorine.

[6] The catalyst for gas-phase catalytic ammoxidation reaction according to [5], wherein a molar ratio of fluorine to molybdenum, F/Mo, is 0.0001 or more and 1 or less.

[7] A method for producing acrylonitrile wherein the catalyst for gas-phase catalytic ammoxidation reaction according to any one of [1] to [6] is used.

[8] A method for producing a catalyst for gas-phase catalytic ammoxidation reaction, the catalyst comprising a metal oxide and a silica carrier carrying the metal oxide, the method comprising:

a preparation step of preparing a precursor slurry comprising a starting material for the silica carrier and Mo;
a drying step of spray drying the precursor slurry to obtain a dried particle; and
a calcination step of calcining the dried particle, and
at least one operation selected from the group consisting of the following (I), (II) and (III) is performed:

(I) adding a fluorine compound in the preparation step and/or the drying step;
(II) keeping a state of a calcination temperature of 695°C or more under an inert gas atmosphere for 3 hours or more in the calcination step; and
(III) by using, as a starting material for the silica carrier, a silica particle having an interaction coefficient C with water vapor of 1 or more and 10 or less, the interaction coefficient C being derived by a water vapor adsorption measurement, adding 50% by mass or more of the silica particle based on a total mass M of the starting material for the silica carrier in the preparation step, wherein the preparation step comprises a first silica particle addition step of adding at least a portion of the starting material for the silica carrier to a mixture comprising Mo, a Nb addition step of adding Nb to the mixture, and a second silica particle addition step of adding at least a portion of a balance of the starting material for the silica carrier to the mixture at the same time as the Nb addition step or after the Nb addition step, and wherein a ratio of a mass M1 of the starting material for the silica carrier to the total mass M, M1/M, in the first silica particle addition step is 0.4 to 0.7.

[9] The method for producing the catalyst for gas-phase catalytic ammoxidation reaction according to [8], wherein the metal oxide is represented by the following formula (1):

$$Mo_1V_aSb_bNb_cW_dX_eO_n \ldots \quad (1)$$

wherein X represents at least one selected from the group consisting of Te, Ce, Ti and Ta; a, b, c, and d satisfy relational expressions of $0.01 \leq a \leq 1$, $0.01 \leq b \leq 1$, $0.01 \leq c \leq 1$, $0 \leq d \leq 1$, and $0 \leq e \leq 1$; and n represents a number determined by valences of the other elements.

[10] The method for producing the catalyst for gas-phase catalytic ammoxidation reaction according to [8], wherein the metal oxide is represented by the following formula (2) :

$$Mo_{12}Bi_fFe_gX_hY_iZ_jO_m \ldots \quad (2)$$

wherein X represents at least one element selected from the group consisting of nickel, cobalt, magnesium, calcium, zinc, strontium and barium; Y represents at least one element selected from the group consisting of cerium, chromium, lanthanum, neodymium, yttrium, praseodymium, samarium, aluminum, gallium and indium; Z represents at least one element selected from the group consisting of potassium, rubidium and cesium; f, g, h, i, j and m represent atomic ratios of the elements, and respectively satisfy $0.1 \leq f \leq 2.0$, $0.1 \leq g \leq 3.0$, $0.1 \leq h \leq 10.0$, $0.1 \leq i \leq 3.0$, and $0.01 \leq j \leq 2.0$; and m represents a number of oxygen atoms necessary for satisfying valence requirements of the other elements present.

[11] The method for producing the catalyst for gas-phase catalytic ammoxidation reaction according to any one of [8] to [10], wherein the fluorine compound is added so that a molar ratio of fluorine to molybdenum, F/Mo, in the precursor slurry is 0.001 or more and 1 or less.

[12] The method for producing the catalyst for gas-phase catalytic ammoxidation reaction according to any one of [8] to [11], wherein the fluorine compound comprises ammonium fluoride.

Advantageous Effects of Invention

According to the present invention, a catalyst for gas-phase catalytic ammoxidation reaction and a method for producing a catalyst for gas-phase catalytic ammoxidation reaction, in which ammonia and hydrocarbon as starting materials can efficiently be utilized and the yield can also be improved, can be provided.

Description of Embodiments

Hereinafter, an embodiment of the present invention (hereinafter, referred to as "present embodiment".) will be described in detail. It is to be noted that the present invention is not limited to the following present embodiment and can be carried out by modifying the present embodiment variously within the scope of the present invention. In a case where numerical values or physical property values are expressed before and after "-" in such a way as to interpose "-" therebetween in the present specification, the values are used on condition that the values before and after "-" are included. For example, the notation of a numerical value range of "1 to 100" includes both of the upper limit value "100" and the lower limit value "1". The same applies to the notations of the other numerical value ranges.

<Catalyst for Gas-Phase Catalytic Ammoxidation Reaction>

A catalyst for gas-phase catalytic ammoxidation reaction of the present embodiment is a catalyst for gas-phase catalytic ammoxidation reaction, the catalyst including a metal oxide and a silica carrier carrying the metal oxide, wherein the metal oxide contains molybdenum (Mo), and an interaction coefficient C between water vapor and the catalyst for gas-phase catalytic ammoxidation reaction, the interaction coefficient C being derived by subjecting the catalyst for gas-phase catalytic ammoxidation reaction to water vapor adsorption measurement, is 1 or more and 30 or less. The catalyst for gas-phase catalytic ammoxidation reaction of the present embodiment is configured as described above, and thus ammonia and hydrocarbon as starting materials can efficiently be utilized and the yield can also be improved.

The catalyst for gas-phase catalytic ammoxidation reaction of the present embodiment is used for gas-phase catalytic ammoxidation reaction of hydrocarbon, and includes a metal oxide and a silica carrier. Hereinafter, the reaction of synthesizing an unsaturated nitrile from hydrocarbon, an oxygen source such as oxygen, and a nitrogen source such as ammonia will simply be referred to as "gas-phase catalytic ammoxidation reaction". It is to be noted that the unsaturated nitrile in the present embodiment is preferably acrylonitrile. The catalyst for gas-phase catalytic ammoxidation reaction of the present embodiment has performance suitable for a catalyst of gas-phase catalytic ammoxidation reaction. The method for evaluating such performance is not limited to the following, but, for example, the catalyst having such performance can be confirmed by subjecting the catalyst to XRD measurement according to an ordinary method and confirming that a predetermined crystal phase (but not limited to, typically, a crystal phase constituted by MoVSbNb or MoBiFe) is present. Additionally, for example, the catalyst having such performance can be confirmed by performing measurement of the yield of acrylonitrile by the catalyst, according to the method described in Examples, which will be mentioned later, and confirming that the yield of acrylonitrile is 30% or more.

(Metal Oxide)

The catalyst for gas-phase catalytic ammoxidation reaction of the present embodiment includes a metal oxide as a catalyst component of the gas-phase catalytic ammoxidation reaction. The metal oxide is not particularly limited as long as it contains Mo, and the composition thereof can appropriately be adjusted with respect to each starting raw material. A preferred composition of the metal oxide will be mentioned later, with respect to each starting raw material.

(Silica Carrier)

The catalyst for gas-phase catalytic ammoxidation reaction of the present embodiment includes a silica carrier carrying the metal oxide, as a carrier of the metal oxide. The silica carrier is not particularly limited as long as it contains silica, and the composition thereof can appropriately be adjusted with respect to each starting raw material. A preferred composition of the silica carrier will be mentioned later, with respect to each starting raw material.

(Content of silica carrier)

In the present embodiment, the mass proportion of the silica carrier in terms of $SiO_2$ based on the total amount of the catalyst for gas-phase catalytic ammoxidation reaction is preferably 30% by mass or more and 70% by mass or less, more preferably 35 to 65% by mass, still more preferably 40 to 60%, by mass from the viewpoint of wear resistance and strength of a catalyst particle.
Examples of the starting material for the silica carrier include, but not particularly limited to, silica sol (also called colloidal silica) and powdery silica (dry silica).

(Interaction coefficient C)

The reason why the interaction coefficient C can be within a predetermined range in the present embodiment to allow for efficient use of ammonia and hydrocarbon as starting materials and also an improvement in yield is inferred by the present inventors, as follows. That is, it is considered that, while ammonia and hydrocarbon (a saturated hydrocarbon such as propane or an unsaturated hydrocarbon such as propylene) as starting materials adsorb to a catalyst surface (active site of the catalyst) and thus reaction progresses in a reaction system in gas-phase catalytic ammoxidation of hydrocarbon, in a case where a catalyst surface including a pore of a catalyst carrier is low in hydrophobicity, adsorption of a water molecule (water vapor) co-existing in the reaction system would likely be caused whereas adsorption of the starting materials to a catalyst surface would be competitively inhibited. The present inventors have here found that a main factor of deterioration in hydrophobicity in a catalyst containing a silica carrier is a silanol group. That is, it is considered that, if a hydrophilic silanol group present in a catalyst surface is decreased, hydrophobicity of the catalyst surface can significantly be increased and adsorption of a starting material can be promoted while adsorption of a water molecule to the catalyst surface is suppressed. More specifically, it is considered that a catalyst surface is high in hydrophobicity to thereby suppress diffusion of water vapor in a catalyst pore and easily remove the generated water vapor from the pore. The present inventors have here found that hydrophobicity of a catalyst surface can properly be evaluated by an interaction coefficient C. That is, it has been found that a numerical value range of the interaction coefficient C is optimized and thus ammonia and hydrocarbon as starting materials can efficiently be utilized and the yield is also be improved.
From the above viewpoint, in the present embodiment, the interaction coefficient C between water vapor and the catalyst for gas-phase catalytic ammoxidation reaction, the interaction coefficient C being derived by subjecting the catalyst for gas-phase catalytic ammoxidation reaction to water vapor adsorption measurement, is 1 or more and 30 or less, preferably 1.5 or more and 17 or less, more preferably 2 or more and 10 or less, and still more preferably 2 or more and less than 10.
The interaction coefficient C can be measured by the method described in Examples, which will be mentioned later.
The water vapor adsorption measurement refers to an analysis where the pressure of water vapor is gradually increased from a state of no adsorption to a solid surface in vacuum and the amount of adsorption of water vapor to the solid surface at each pressure is measured. The interaction coefficient C represents the difference in heat of adsorption between a first layer and a second or subsequent layer of adsorption, meaning that, as the value is higher, the solid surface has stronger interaction with water vapor, namely, the solid surface is more hydrophilic. Thus, the interaction coefficient C can be adjusted to the above-described range by, for example, increasing hydrophobicity of the catalyst surface according to a preferred production method mentioned later.

The catalyst for gas-phase catalytic ammoxidation reaction of the present embodiment preferably further includes fluorine from the viewpoint of a more increase in hydrophobicity of the catalyst surface. The state of the presence of fluorine is not particularly limited, and fluorine may be exchanged with OH of silanol in the silica carrier and then present, or may be bound or coordinated with a metal in the metal oxide.

The molar ratio of fluorine to molybdenum, F/Mo, in the catalyst for gas-phase catalytic ammoxidation reaction of the present embodiment is preferably 0.0001 or more and 1 or less. When the molar ratio is 0.0001 or more, there is a tendency that hydrophobicity of the catalyst surface is more increased, and when the molar ratio is 1 or less, there is a tendency that the influence of the catalyst on crystal formation can sufficiently be suppressed to result in more improvements in reaction results. From the viewpoint similar to that described above, the molar ratio is more preferably 0.0005 to 0.5, and still more preferably 0.001 to 0.1.

The molar ratio can be measured by the method described in Examples, which will be mentioned later.

The molar ratio can be adjusted in the above-described range by fluorine addition or the like in catalyst production.

The specific surface area of the catalyst for gas-phase catalytic ammoxidation reaction of the present embodiment is not particularly limited, but is preferably 5 to 70 $m^2/g$. The specific surface area can be measured with an automatic specific surface area measurement apparatus Gemini V manufactured by Micromeritics Instrument Corp., according to a BET 1-point method, by preliminarily drying a sample under a helium flow at 300°C for 15 minutes and then using nitrogen as an adsorption gas.

The proportion of a particle having a particle diameter of 45 $\mu$m or less in a particle size distribution on a volume basis of the catalyst for gas-phase catalytic ammoxidation reaction of the present embodiment is not particularly limited, but is preferably 5 to 45% (the accumulation of 45 $\mu$m in a volume basis distribution: 5 to 450). The proportion can be obtained as the proportion of a particle having a particle diameter of 45 $\mu$m or less (accumulation of 45 $\mu$m in a volume basis distribution), from a particle size distribution on a volume basis obtained by adopting water as a dispersion medium, placing 0.6 g of the catalyst for gas-phase catalytic ammoxidation reaction in 250 mL of water, performing an ultrasonic dispersion treatment for one minute, and then performing measurement under a condition of a relative refractive index of 1.40 with a laser diffraction/scattering particle size distribution measurement apparatus LA-300 manufactured by Horiba Ltd.

The shape of the catalyst for gas-phase catalytic ammoxidation reaction of the present embodiment is not particularly limited, but is preferably spherical in consideration of fluidity in the case of use as a fluidized bed catalyst. The median diameter of the catalyst for gas-phase catalytic ammoxidation reaction of the present embodiment is not particularly limited, but is preferably 10 to 180 $\mu$m, more preferably 10 to 150 $\mu$m, and still more preferably 20 to 150 $\mu$m. The median diameter can be obtained by adopting water as a dispersion medium, placing 0.6 g of the catalyst in 250 mL of water, performing an ultrasonic dispersion treatment for one minute, and then performing measurement under a condition of a relative refractive index of 1.40 with a laser diffraction/scattering particle size distribution measurement apparatus LA-300 manufactured by Horiba Ltd.

The apparent specific gravity of the catalyst for gas-phase catalytic ammoxidation reaction of the present embodiment is not particularly limited, but is preferably 0.8 to 1.2 g/cc. The apparent specific gravity is determined by using a funnel and a 25-cc measuring cylinder and dropping the catalyst from the funnel toward the measuring cylinder, levelling the catalyst on the upper portion of the measuring cylinder by a metallic ruler or the like, metering the weight of the measuring cylinder, and subtracting the tare weight of the measuring cylinder to thereby obtain the weight of the catalyst. The apparent specific gravity can be calculated by the following expression with the obtained weight of the catalyst.

Apparent specific gravity = weight of catalyst (g)/25 (cc)

The catalyst for gas-phase catalytic ammoxidation reaction of the present embodiment can be applied to both gas-phase catalytic ammoxidation reaction of a saturated hydrocarbon and gas-phase catalytic ammoxidation reaction of an unsaturated hydrocarbon, and the composition, physical properties and the like thereof can appropriately be adjusted with respect to each application. Hereinafter, the former is defined as a first aspect and the latter is defined as a second aspect, and these aspects will be described with respect to each application.

[First Aspect]

The catalyst for gas-phase catalytic ammoxidation reaction of the present embodiment is used for gas-phase catalytic ammoxidation reaction of a saturated hydrocarbon such as propane in the first aspect. In such an application, the metals in the metal oxide preferably contain not only Mo, but also vanadium (V), antimony (Sb), and niobium (Nb), and may, if necessary, contain an additional metal.

More specifically, the metal oxide in the catalyst for gas-phase catalytic ammoxidation reaction in the first aspect is preferably represented by the following formula (1):

$$Mo_1V_aSb_bNb_cW_dX_eO_n \ldots \quad (1)$$

wherein X represents at least one selected from the group consisting of Te, Ce, Ti and Ta; a, b, c, and d satisfy relational expressions of $0.01 \leq a \leq 1$, $0.01 \leq b \leq 1$, $0.01 \leq c \leq 1$, $0 \leq d \leq 1$, and $0 \leq e \leq 1$; and n represents a number determined by valences of the other elements.

[Second Aspect]

The catalyst for gas-phase catalytic ammoxidation reaction of the present embodiment is used for gas-phase catalytic ammoxidation reaction of an unsaturated hydrocarbon such as propylene in the second aspect. In such an application, the metals in the metal oxide preferably contain not only Mo, but also bismuth (Bi) and iron (Fe), and may if necessary, contain an additional metal.

More specifically, the metal oxide in the catalyst for gas-phase catalytic ammoxidation reaction in the second aspect is preferably represented by the following formula (2):

$$Mo_{12}Bi_fFe_gX_hY_iZ_jO_m \ldots \quad (2)$$

wherein X represents at least one element selected from the group consisting of nickel, cobalt, magnesium, calcium, zinc, strontium and barium; Y represents at least one element selected from the group consisting of cerium, chromium, lanthanum, neodymium, yttrium, praseodymium, samarium, aluminum, gallium and indium; Z represents at least one element selected from the group consisting of potassium, rubidium and cesium; f, g, h, i, j and m represent atomic ratios of the elements, and respectively satisfy $0.1 \leq f \leq 2.0$, $0.1 \leq g \leq 3.0$, $0.1 \leq h \leq 10.0$, $0.1 \leq i \leq 3.0$, and $0.01 \leq j \leq 2.0$; and m represents the number of oxygen atoms necessary for satisfying valence requirements of the other elements present.

<Method for Producing Catalyst for Gas-Phase Catalytic Ammoxidation Reaction>

In the present embodiment, a method for producing a catalyst for gas-phase catalytic ammoxidation reaction (hereinafter, also referred to as "production method of the present embodiment".) is a method for producing a catalyst for gas-phase catalytic ammoxidation reaction, the catalyst including a metal oxide and a silica carrier carrying the metal oxide, the method including a preparation step of preparing a precursor slurry including a starting material for the silica carrier and Mo, a drying step of spray drying the precursor slurry to obtain a dried particle, and a calcination step of calcining the dried particle, and at least one operation selected from the group consisting of the following (I), (II) and (III) is performed:

(I) adding a fluorine compound in the preparation step and/or the drying step;
(II) keeping a state of a calcination temperature of 695°C or more under an inert gas atmosphere for 3 hours or more in the calcination step; and
(III) by using, as a starting material for the silica carrier, a silica particle having an interaction coefficient C with water vapor of 1 or more and 10 or less, the interaction coefficient C being derived by a water vapor adsorption measurement, adding 50% by mass or more of the silica particle based on a total mass M of the starting material for the silica carrier in the preparation step, wherein the preparation step includes a first silica particle addition step of adding at least a portion of the starting material for the silica carrier to a mixture comprising Mo, a Nb addition step of adding Nb to the mixture, and a second silica particle addition step of adding at least a portion of the balance of the starting material for the silica carrier to the mixture at the same time as the Nb addition step or after the Nb addition step, and wherein a ratio of a mass M1 of the starting material for the silica carrier to the total mass M, M1/M, in the first silica particle addition step is 0.4 to 0.7. That is, the catalyst for gas-phase catalytic ammoxidation reaction of the present embodiment is more preferably obtained by the production method of the present embodiment.

(Preparation step)

The production method of the present embodiment includes a preparation step of preparing a precursor slurry including a starting material for the silica carrier and Mo, as described above. The precursor slurry is not particularly limited as long as it includes the starting material for the silica carrier and Mo, and the slurry composition of the balance can appropriately be adjusted with respect to each starting raw material. A preferred method for producing the precursor slurry will be mentioned later, with respect to each starting raw material.

(Drying step)

The production method of the present embodiment includes a drying step of spray drying the precursor slurry to obtain a dried particle as described above. A drying condition is not particularly limited, and can appropriately be adjusted with respect to each starting raw material. A specific drying condition will be mentioned later, with respect to each starting raw material.

(Calcination step)

The production method of the present embodiment includes a calcination step of calcining the dried particle, as described above. A calcination condition is not particularly limited, and can appropriately be adjusted with respect to each starting raw material. A specific calcination condition will be mentioned later, with respect to each starting raw material.

(Hydrophobization of catalyst surface)

The above (I) can be performed for hydrophobization of the catalyst surface in the production method of the present embodiment. That is, hydrophobization of the catalyst surface can be promoted by addition of a fluorine compound in the preparation step and/or the drying step. The above (I) can be performed in the drying step instead of implementation in the preparation step or in addition to implementation in the preparation step. When the above (I) is performed in the preparation step, a fluorine compound may be added to the precursor slurry. When the above (I) is performed in the drying step, for example, a fluorine compound-containing solution may be sprayed to a resultant dried particle, or a resultant dried particle may be immersed in a fluorine compound-containing solution. By performing the above (I), an OH moiety of silanol on a silica carrier surface is substituted with a fluorine anion to result in an increase in hydrophobicity.

The fluorine compound is preferably added in the operation (I) above so that the molar ratio of fluorine to molybdenum in the precursor slurry, as F/Mo, is 0.001 or more and 1 or less. When the molar ratio is 0.001 or more, there is a tendency that hydrophobicity of the catalyst surface is more increased, and when the molar ratio is 0.1 or less, there is a tendency that the influence of the catalyst on crystal formation can sufficiently be suppressed to result in more improvements in reaction results. From the viewpoint similar to that described above, the molar ratio is more preferably 0.002 to 0.5, and still more preferably 0.005 to 0.1.

Examples of the fluorine compound in the operation (I) include, but not particularly limited to, ammonium fluoride, calcium fluoride, aluminum fluoride, sodium fluoride, cerium fluoride, trifluoroacetic acid, and trifluoroaniline. Among these, ammonium fluoride is preferable from the viewpoint of catalyst performance.

The above (II) can be performed, instead of the above (I) or in addition to the above (I), for hydrophobization of the catalyst surface in the production method of the present embodiment. That is, hydrophobization of the catalyst surface can be promoted by keeping a state of a calcination temperature of 695°C or more under an inert gas atmosphere for 3 hours or more in the calcination step. Such calcination conditions can allow for a decrease of silanol on a silica carrier surface and a significant increase in hydrophobicity.

The above (III) can be performed, instead of the above (I) and (II) or in addition to the above (I) and (II), for hydrophobization of the catalyst surface in the production method of the present embodiment. By using, as the starting material for the silica carrier, a silica particle (hereinafter, also referred to as "dry silica".) having an interaction coefficient C with water vapor of 1 or more and 10 or less, the interaction coefficient C being derived by the water vapor adsorption measurement, 50% by mass or more of the silica particle based on the total mass of the starting material for the silica carrier is added in the preparation step, wherein the preparation step includes a first silica particle addition step of adding at least a portion of the starting material for the silica carrier to a mixture comprising Mo, a Nb addition step of adding Nb to the mixture, and a second silica particle addition step of adding at least a portion of the balance of the starting material for the silica carrier to the mixture at the same time as the Nb addition step or after the Nb addition step, and wherein the ratio of the mass M1 of the starting material for the silica carrier to the total mass M, M1/M, in the first silica particle addition step is 0.4 to 0.7. Thus, the proportion of a silica surface for reaction with Nb can be maintained at a certain or higher level in Nb starting material addition. Thus, a silanol group on the silica surface reacts with Nb, thereby allowing for a decrease of silanol on a silica carrier surface and a significant increase in hydrophobicity. Furthermore, Nb being an element to be easily aggregated is bound to the silica surface with retaining dispersibility, and thus the dispersibility of Nb in a catalytically active species can be improved and the effect of improving catalyst performance is also exerted.

The interaction coefficient C of the dry silica can be measured by the method described in Examples, which will be mentioned later, and can be adjusted in the above-described range by use of dry silica treated at a sufficiently high temperature. The amount of addition of the dry silica is 50% by mass or more, more preferably 55% by mass or more, and still more preferably 60% by mass or more.

Any commercially available one can be adopted as the dry silica in the present embodiment, and examples thereof include, but not limited to, AEROSIL 90, AEROSIL 130, AEROSIL 150, AEROSIL 255, AEROSIL 200, AEROSIL 300, AEROSIL 380, and AEROSIL OX 50 (all are powders).

When the dry silica is adopted as a starting material in the present embodiment, an aqueous dispersion liquid of the dry silica may also be used. Such an aqueous dry silica dispersion liquid may be used in the form of the dry silica dispersed in water. Any commercially available one can also be adopted as such an aqueous dry silica dispersion liquid, and specific examples thereof include, but not limited to, AERODISP W 1244, AERODISP W 630, AERODISP W 7330N, AERODISP W 740X, AERODISP W 7520 N, and AERODISP W 7622 (all are aqueous dry silica dispersion liquids) (all are manufactured by NIPPON AEROSIL Co., Ltd.).

The starting material for the silica carrier other than the dry silica is, for example, a silica particle having an interaction coefficient C with water vapor of more than 10 as derived by water vapor adsorption measurement, or a silica particle from which interaction coefficient C cannot be obtained. Examples of the silica particle having an interaction coefficient C of more than 10 or the silica particle, from which interaction coefficient C cannot be obtained, include silica sol with water as a solvent. The silica sol is produced by a wet method, and thus is remarkably high in silanol group density in a surface thereof as compared with a dry silica powder. The silica sol is produced and used in the form of being dispersed in an aqueous solvent, and thus the interaction coefficient C as an analysis procedure of a powder cannot be measured.

The ratio of the mass M1 of the starting material for the silica carrier to the total mass M in the first silica particle addition step, as M1/M, is 0.4 to 0.7. This range can allow for an increase in hydrophobicity of the catalyst surface. The M1/M is preferably 0.45 to 0.65, more preferably 0.5 to 0.6.

The addition of the dry silica in the preparation step in the production method of the present embodiment is preferably divided to two or more portions. When the dry silica is added in two portions, the first addition is preferably made at the same time as addition of the silica sol and the second addition is preferably made at the same time as or after the subsequent addition of the Nb starting material.

If necessary, the production method of the present embodiment may include a removal step of removing a protrusion of the obtained catalyst.

The production method of the present embodiment can be applied to both production of a catalyst for gas-phase catalytic ammoxidation reaction according to the first aspect and production of a catalyst for gas-phase catalytic ammoxidation reaction according to the second aspect, and a condition of each step with respect to each of the applications can appropriately be adjusted. Hereinafter, the production method of the present embodiment will be described with respect to each of the aspects.

[First Aspect]

The catalyst for gas-phase catalytic ammoxidation reaction according to the first aspect can preferably be produced by the production method of the present embodiment. That is, a catalyst for gas-phase catalytic ammoxidation reaction, including a metal oxide represented by the following formula (1), can preferably be produced by the production method of the present embodiment. A typical condition for producing the catalyst for gas-phase catalytic ammoxidation reaction according to the first aspect will be described later.

It is to be noted that at least one operation selected from the group consisting of the above (I), (II) and (III) is performed for producing the catalyst for gas-phase catalytic ammoxidation reaction according to the first aspect and, if a selected condition and the following condition are different, the above-mentioned operation is preferentially adopted.

(Preparation Step)

The preparation step is not particularly limited, and, for example, includes: preparation step A of mixing a molybdenum starting material, a vanadium starting material, an antimony starting material, and water, thereby preparing aqueous mixed liquid A; preparation step B of mixing a niobium starting material and an organic acid, thereby preparing niobium starting material liquid B; and mixing step C of mixing aqueous mixed liquid A and niobium starting material liquid B, thereby preparing precursor slurry C.

The catalyst for gas-phase catalytic ammoxidation reaction according to the first aspect can be produced by adopting a preferred preparation condition, which will be mentioned later, in preparation step B or by adopting a preferred mixing condition, which will be mentioned later, in mixing step C.

Next, niobium starting material liquid B containing an organic acid and a niobium starting material and aqueous mixed liquid A, which have been prepared in preparation step B in advance, are mixed to match the intended composition, thereby obtaining a slurry as a precursor slurry (mixing step C). For example, in a case where the catalyst contains W or Ce, the precursor slurry is obtained by suitably mixing a compound containing W. The compound containing W or Ce can be added in aqueous mixed liquid A, or can be added simultaneously in mixing niobium starting material liquid B and aqueous mixed liquid A.

Silica sol can timely be added in preparation of the precursor slurry.

(Preparation Step A)

Preparation step A is a step of mixing a molybdenum starting material, a vanadium starting material, an antimony starting material, and water, thereby preparing aqueous mixed liquid A. More specifically, the molybdenum starting material, the vanadium starting material, and the antimony starting material are added to water and heated at a predetermined temperature or higher under stirring, and aqueous mixed liquid A can thereby be prepared. On this occasion, in a case where the catalyst contains W or Ce, a tungsten starting material or a cerium starting material may further be added.

The component starting materials to be used for aqueous mixed liquid A are not particularly limited, and for example, the following compounds can be used.

Examples of the molybdenum starting material include, but not particularly limited to, molybdenum oxide, ammonium dimolybdate, ammonium heptamolybdate, phosphomolybdic acid, and silicomolybdic acid, and among others, ammonium heptamolybdate can suitably be used.

Examples of the vanadium starting material include, but not particularly limited to, vanadium pentoxide, ammonium metavanadate, and vanadyl sulfate, and among others, ammonium metavanadate can suitably be used.

As the antimony starting material, an oxide of antimony can suitably be used. The tungsten starting material is not particularly limited, and for example, ammonium metatungstate is suitably used. The cerium starting material is not particularly limited, and for example, cerium nitrate hexahydrate is suitably used.

The lower limit of the temperature during stirring is usually 80°C or higher, and is preferably 90°C or higher. There is a tendency that when the temperature during stirring is set to 80°C or higher, the oxidation/reduction reaction between the oxide of antimony, which is, in general, hardly soluble to water, and another oxide starting material (for example, vanadium starting material) is more facilitated. On the other hand, it is preferable that the upper limit of the temperature during stirring be usually 100°C or lower in order to avoid bumping.

(Preparation Step B)

Preparation step B is a step of mixing a niobium starting material and an organic acid, thereby preparing niobium starting material liquid B. In preparation step B, water may further be mixed. The niobium starting material is hardly soluble in general and is therefore dissolved in water by allowing the organic acid to coexist. On this occasion, it is preferable that the Nb content in niobium starting material liquid B be high, and the dispersibility of Nb be satisfactory in the preparation of the catalyst for gas-phase catalytic ammoxidation reaction according to the first aspect.

The niobium starting material is not particularly limited as long as it is a compound containing Nb, but any of such compounds is hardly soluble and therefore is dissolved by allowing the organic acid to coexist. Specific examples of the niobium starting material include, but not limited to, niobium hydrogen oxalate, ammonium niobium oxalate, $NbCl_3$, $NbCl_5$, $Nb_2(C_2O_4)_5$, $Nb_2O_5$, niobic acid, $Nb(OC_2H_5)_5$, a halide of niobium, a halogenated ammonium salt of niobium, and combinations thereof. Among these, in a case where an additional metal is added to niobium starting material liquid B, niobic acid and niobium hydrogen oxalate are preferable from the viewpoint of reducing an influence on the additional metal. It is to be noted that niobic acid contains niobium hydroxide and niobium oxide. The niobium starting material changes in quality in some cases due to long-term storage or progress of dehydration, and therefore a niobium starting material immediately after production is preferably used for preparing niobium starting material liquid B, but a compound which has somewhat undergone a change in quality may be used.

The niobium starting material may be a solid or may take the form of suspension in preparing niobium starting material liquid B. In a case where niobic acid is used, the particle diameter is preferably smaller from the viewpoint of easiness of dissolution. Niobic acid can be washed with ammonia water and/or water before use.

Examples of the organic acid include, but not particularly limited to, a dicarboxylic acid. Specific examples of the dicarboxylic acid include oxalic acid, malonic acid, succinic acid, and glutaric acid, and from the viewpoint of suppressing over-reduction of the metal oxide in the calcination stage at the time of producing the catalyst, oxalic acid is preferable, more specifically, an oxalic anhydride and oxalic acid dihydrate are preferable. Only one dicarboxylic acid may be added, or a plurality of dicarboxylic acids may be combined.

(Mixing Step C)

Mixing step C is a step of mixing aqueous mixed liquid A and niobium starting material liquid B, thereby preparing precursor slurry C. In mixing step C, if necessary, hydrogen peroxide, a basic aqueous solution, an additional metal starting material such as a tungsten starting material or a cerium starting material, and/or a carrier starting material may further be mixed. Precursor slurry C which is obtained in this way is a uniform mixed liquid in some cases, but is usually a slurry.

The mixing condition in mixing step C is not particularly limited, but, for example, the mixing temperature is preferably 40°C or higher and 80°C or lower, and more preferably 50°C or higher and 80°C or lower. The stirring time is preferably 1 hour or longer and 5 hours or shorter.

Examples of the method of adding hydrogen peroxide in mixing step C include: a method in which aqueous mixed liquid A and hydrogen peroxide are mixed and then further niobium starting material liquid B is mixed; a method in which niobium starting material liquid B and hydrogen peroxide are mixed and then further aqueous mixed liquid A is mixed; a method in which aqueous mixed liquid A, niobium starting material liquid B, and hydrogen peroxide are simultaneously mixed; and a method in which aqueous mixed liquid A and niobium starting material liquid B are mixed, and then further hydrogen peroxide is mixed. Among these, the method in which aqueous mixed liquid A and hydrogen peroxide are mixed and then further niobium starting material B is mixed is preferable.

In a case where hydrogen peroxide is added in mixing step C, the molar ratio of hydrogen peroxide to antimony (Sb) ($H_2O_2$/Sb) in aqueous mixed liquid A is preferably 0.01 or more and 5.0 or less, more preferably 2.0 or more and 4.0 or less, and still more preferably 2.5 or more and 3.5 or less. In a case where aqueous mixed liquid A and hydrogen peroxide are mixed, it is preferable to add hydrogen peroxide while stirring aqueous mixed liquid A under a heating condition. On this occasion, the temperature is usually 30°C to 70°C, preferably 60°C or higher. The stirring time is preferably 30 minutes to 2 hours.

Examples of the method of adding the basic aqueous solution in mixing step C include: a method in which aqueous mixed liquid A and the basic aqueous solution are mixed and then further niobium starting material liquid B is mixed; a method in which niobium starting material liquid B and the basic aqueous solution are mixed and then further aqueous mixed liquid A is mixed; a method in which aqueous mixed liquid A, niobium starting material liquid B, and the basic aqueous solution are simultaneously mixed; and a method in which aqueous mixed liquid A and niobium starting material liquid B are mixed and then further the basic aqueous solution is mixed. Among these, the method in which aqueous mixed liquid A and niobium starting material liquid B are mixed and then further the basic aqueous solution is mixed is preferable.

Examples of the basic aqueous solution to be added in mixing step C include, but not particularly limited to, ammonia water, an amine, and an alkali aqueous solution. Among these, the basic aqueous solution is most preferably ammonia water because the ammonia water evaporates for the most part in the drying step and does not affect the steps after the drying step.

In a case where the basic aqueous solution is added in mixing step C, the molar ratio of $NH_3$ to niobium (Nb) in niobium starting material liquid B ($NH_3$/Nb) is preferably 0.01 or more and 5 or less, more preferably 2.0 or more and 4 or less, and still more preferably 2.5 or more and 3.5 or less.

Among those described above, it is preferable to mix hydrogen peroxide in such a way that the molar ratio of hydrogen peroxide to antimony (Sb) ($H_2O_2$/Sb) is 2.5 or more and to mix ammonia in such a way that the molar ratio of ammonia to niobium (Nb) ($NH_3$/Nb) is 2.0 or more in mixing step C from the viewpoint of more efficiently obtaining the catalyst for gas-phase catalytic ammoxidation reaction according to the first aspect. In addition, it is preferable to set pH in mixing step C to 5 or more and to obtain the precursor slurry by performing stirring under a heating condition of 60°C or higher. The condition that satisfies these is referred to as mixing condition c. It is to be noted that pH can be adjusted using the above-mentioned basic aqueous solution.

Examples of the method of adding an additional metal starting material such as a tungsten starting material or a cerium starting material in mixing step C include: a method in which aqueous mixed liquid A and the additional metal starting material are mixed and then further niobium starting material liquid B is mixed; a method in which niobium starting material liquid B and the additional metal starting material are mixed and then further aqueous mixed liquid A is mixed; a method in which aqueous mixed liquid A, niobium starting material liquid B, and the additional metal starting material are simultaneously mixed; and a method in which aqueous mixed liquid A and niobium starting material liquid B are mixed and then further the additional metal starting material is mixed.

Examples of the method of adding the carrier starting material in mixing step C include: a method in which aqueous mixed liquid A and the carrier starting material are mixed and then further niobium starting material liquid B is mixed; a method in which niobium starting material liquid B and the carrier starting material are mixed and then further aqueous mixed liquid A is mixed; a method in which aqueous mixed liquid A, niobium starting material liquid B, and the carrier starting material are simultaneously mixed; a method in which aqueous mixed liquid A and niobium starting material liquid B are mixed and then further the carrier starting material is mixed; and a method as any combination of the above-mentioned methods, in which the carrier starting material is mixed in a plurality of portions.

(Drying Step)

The drying step is a step of drying precursor slurry C obtained in the above-mentioned step, thereby obtaining a dried powder. The drying can be performed by a known method, and can be performed by, for example, spray drying or evaporation to dryness. Among these, a fine spherical dried particle is preferably obtained by spray drying. Nebulization in the spray drying method can be performed by a centrifugal system, a two-fluid nozzle system, or a high-pressure nozzle system. As the heat source for drying, steam, or air heated with an electric heater or the like can be used. The inlet temperature of a drier in a spray drying apparatus is preferably 150 to 300°C, and the outlet temperature of the drier is preferably 100 to 160°C.

(Calcination Step)

The calcination step is a step of calcining the dried powder obtained in the drying step, thereby obtaining the catalyst for gas-phase catalytic ammoxidation reaction. As a calcination apparatus, a rotary furnace (rotary kiln) can be used. The shape of a calciner is not particularly limited, but is preferably a tubular shape because continuous calcination can be carried out. The shape of a calcination pipe is not particularly limited, but is preferably cylindrical. As a heating system, an external heating type is preferable, and an electric furnace can suitably be used.

Appropriate size, material, and the like of a calcination pipe can be selected according to the calcination condition or the production amount, but the inner diameter of the calcination pipe is preferably 70 to 2000 mm, more preferably 100 to 1200 mm, and the length of the calcination pipe is preferably 200 to 10000 mm, and more preferably 800 to 8000 mm. In a case where shock is given to the calciner, the thickness of the calciner is preferably 2 mm or more, and more preferably 4 mm or more from the viewpoint of having a sufficient thickness to such an extent that the calciner is not broken by the shock, and is preferably 100 mm or less, and more preferably 50 mm or less from the viewpoint of sufficiently transmitting the shock to the inside of the calciner. The material of the calciner is not particularly limited, except that the material has heat resistance and strength such that the material is not broken by the shock, and SUS can suitably be used.

The calcination pipe can be divided into two or more zones by installing, in the calcination pipe, a weir plate vertically to the flow of a powder, the weir plate having a hole for allowing the powder to pass in the central portion. Installing the weir plate makes it easy to secure the retention time in the calcination pipe. The number of the weir plates may one or plural. The material of the weir plate is preferably a metal, and the same material as that of the calcination pipe can suitably be used. The height of the weir plate can be adjusted according to the retention time to be secured. For example, in a case where a powder is supplied at 250 g/hr in a rotary furnace having a SUS calcination pipe with an inner diameter of 150 mm and a length of 1150 mm, the weir plate is preferably 5 to 50 mm, more preferably 10 to 40 mm, and still more preferably 13 to 35 mm. The thickness of the weir plate is not particularly limited and is preferably adjusted according to the size of the calcination pipe. For example, in the case of a rotary furnace having a SUS calcination pipe with an inner diameter of 150 mm and a length of 1150 mm, the thickness of the calcination pipe is preferably 0.3 mm or more and 30 mm or less, and more preferably 0.5 mm or more and 15 mm or less.

The calcination pipe is preferably rotated in order to prevent crazing, cracking, and the like of the dried powder and to perform calcination uniformly. The rotation speed of the calcination pipe is preferably 0.1 to 30 rpm, more preferably 0.5 to 20 rpm, and still more preferably 1 to 10 rpm.

When the dried powder is calcined, it is preferable that raising the temperature for heating the dried powder be started from a temperature lower than 400°C, and the temperature be raised continuously or intermittently up to a temperature within a range of 550 to 800°C.

The calcination atmosphere may be under an air atmosphere or under an air flow, but it is preferable to carry out at least part of the calcination while allowing an inert gas, such as nitrogen, which is substantially free of oxygen, to flow. The supply amount of the inert gas is 50 N litters or more, preferably 50 to 5000 N litters, and still more preferably 50 to 3000 N litters, per kg of the dried powder (N litters mean litters measured under the standard temperature/pressure condition, namely litters measured at 0°C and 1 atm). On this occasion, there is no problem when the inert gas and the dried powder are brought into contact in a parallel flow or a counter flow, but counterflow contact is preferable in consideration of gas components generated from the dried powder and the air which is mixed in a slight amount together with the dried powder.

The calcination step can be carried out in one stage, but it is preferable that the calcination include preliminary calcination and final calcination, the preliminary calcination be performed in a temperature range of 250 to 400°C, and the final calcination be performed in a temperature range of 550 to 800°C. The preliminary calcination and the final calcination may be carried out continuously, or after the preliminary calcination is completed, the final calcination may be carried out anew. In addition, each of the preliminary calcination and the final calcination may be divided into several stages.

The preliminary calcination is performed at a heating temperature in a range of 250°C to 400°C, and preferably 300°C to 400°C, preferably in an inert gas flow. The temperature is preferably held at a constant temperature in a temperature range of 250°C to 400°C, but it does not matter if the temperature varies, or gently rises or decreases in the range of 250°C to 400°C. The time for holding the heating temperature is preferably 30 minutes or longer, and more preferably 3 to 12 hours.

With respect to the temperature raising pattern until the time when the temperature reaches a preliminary calcination temperature, the temperature may be raised linearly, or the temperature may be raised in such a way as to draw an upward or downward convex arc.

The average temperature raising rate during raising temperature until the temperature reaches the preliminary calcination temperature is not particularly limited, but is generally about 0.1 to 15°C/min, preferably 0.5 to 5°C/min, and more preferably 1 to 2°C/min.

The final calcination is carried out at 550 to 800°C, preferably 580 to 750°C, more preferably 600 to 720°C, and still more preferably 620 to 700°C, preferably in an inert gas flow. The temperature is preferably held at a constant temperature in a temperature range of 620 to 700°C, but it does not matter if the temperature varies, or gently rises or decreases in the range of 620 to 700°C. The time for the final calcination is 0.5 to 20 hours, and preferably 1 to 15 hours.

In a case where the calcination pipe is divided with the weir plate or plates, the dried powder and/or the composite catalyst for gas-phase catalytic ammoxidation reaction consecutively pass through at least two, preferably 2 to 20, and more preferably 4 to 15 zones. The temperature control can be performed using one or more controllers, but the temperature is preferably controlled by installing a heater and a controller for every zone divided by the weir plates in order to obtain the desired calcination pattern. For example, in a case where seven weir plates are installed in such a way as to equally divide the length of a portion, which enters a heating furnace, of the calcination pipe into eight, and the calcination pipe divided into eight zones is used, the setting temperatures of the eight zones are preferably controlled with the heater and controller installed for the respective zones in such a way that the temperature of the dried powder and/or the composite catalyst for gas-phase catalytic ammoxidation reaction shows the desired calcination temperature pattern. It is to be noted that it does not matter if an oxidative component (for example, oxygen) or a reductive component (for example, ammonia) is added as desired to the calcination atmosphere in an inert gas flow.

With respect to the temperature raising pattern until the time when the temperature reaches a final calcination temperature, the temperature may be raised linearly, or the temperature may be raised in such a way as to draw an upward or downward convex arc.

The average temperature raising rate during raising temperature until the temperature reaches the final calcination temperature is not particularly limited, but is generally 0.1 to 15°C/min, preferably 0.5 to 10°C/min, and more preferably 1 to 8°C/min.

The average temperature decreasing rate after the completion of the final calcination is preferably 0.05 to 100°C/min, and more preferably 0.1 to 50°C/min. Holding the temperature temporarily at a temperature lower than the final calcination temperature is also preferable. The holding temperature is a temperature lower than the final calcination temperature by 10°C, preferably 50°C, and more preferably 100°C. The holding time is 0.5 hours or longer, preferably 1 hour or longer, more preferably 3 hours or longer, and still more preferably 10 hours or longer.

In a case where after the preliminary calcination is completed, the final calcination is carried out anew, a low-temperature treatment is preferably performed in the final calcination.

The time required for the low-temperature treatment, that is the time required until the temperature of the dried powder and/or the composite catalyst for gas-phase catalytic ammoxidation reaction is raised to the calcination temperature after the temperature is lowered can appropriately be adjusted by the size, thickness, and material of the calciner, the production amount of the catalyst, a series of periods of continuously calcining the dried powder and/or the composite catalyst for gas-phase catalytic ammoxidation reaction, the fixing rate/fixing amount, and the like. For example, in a case where a SUS calcination pipe with an inner diameter of 500 mm, a length of 4500 mm, and a thickness of 20 mm is used, the time required for the low-temperature treatment is preferably within 30 days, more preferably within 15 days, still more preferably within 3 days, and particularly preferably within 2 days during the series of periods of continuously calcining the catalyst.

For example, in a case where the dried powder is supplied at a rate of 35 kg/hr while the dried powder is being rotated at 6 rpm with a rotary furnace having a SUS calcination pipe with an inner diameter of 500 mm, a length of 4500 mm, and a thickness of 20 mm, and the final calcination temperature is set to 645°C, a step of lowering the temperature to 400°C and then raising the temperature to 645°C can be performed in about one day. In a case where the calcination is performed for one year continuously, by carrying out such a low-temperature treatment with a frequency of once per month, the calcination can be performed while the oxide layer temperature is being kept stable.

[Second Aspect]

The catalyst for gas-phase catalytic ammoxidation reaction according to the second aspect can preferably be produced by the production method of the present embodiment. That is, a catalyst for gas-phase catalytic ammoxidation reaction, including a metal oxide represented by the following formula (2), can preferably be produced by the production method of the present embodiment. A typical condition for producing the catalyst for gas-phase catalytic ammoxidation reaction according to the second aspect will be described later. It is to be noted that at least one operation selected from the group consisting of the above (I), (II) and (III) is performed for producing the catalyst for gas-phase catalytic ammoxidation reaction according to the second aspect and, if a selected condition and the following condition are different, the above-mentioned operation is preferentially adopted.

The catalyst for gas-phase catalytic ammoxidation reaction according to the second aspect can be produced by a production method including a preparation step of preparing a precursor slurry containing molybdenum, bismuth, iron, and nickel, a drying step of spray drying the precursor slurry to obtain a dried particle, and a calcination step of calcining the dried particle in air to obtain a calcined particle.

(Preparation Step)

Such slurry containing molybdenum, bismuth, and iron is obtained by mixing starting materials for the catalyst and a solvent in the preparation step. The solvent is preferably water, and the slurry is preferably an aqueous slurry. A preparation method is preferably used for carrying the metal oxide by silica, the method including mixing and stirring an aqueous solution containing molybdenum with an aqueous solution containing silica, and then mixing and stirring a solution containing bismuth, iron and other metal.

Silica sol may be used as a starting material for silica from the viewpoint of ease of production. A preferred concentration of silica sol in the state of a starting material not mixed with any additional metal component is 10 to 50% by mass.

The starting material for each element constituting the catalyst, such as molybdenum, bismuth, cerium, iron, nickel, cobalt, magnesium, zinc, potassium, rubidium, and cesium for preparation of the slurry, may be any salt soluble in water or nitric acid, and examples include an ammonium salt, nitrate, hydrochloride, sulfate and an organic acid salt of such each metal. An ammonium salt is suitably used as a starting material containing molybdenum, and nitrate is suitably used as each starting material containing bismuth, cerium, iron, nickel, magnesium, zinc, potassium, rubidium, and cesium.

(Drying Step)

Such slurry containing molybdenum, bismuth, and iron is spray dried in the drying step, and a dried particle is obtained. The slurry is spray dried and a spherical particle is obtained in the spray drying. The spray of aqueous slurry can be performed by a centrifugal system, a two-fluid nozzle system, or a high-pressure nozzle system usually industrially used, and is preferably performed by a centrifugal system. The drying is preferably made by use of air heated, and examples of the heat source for the drying include water vapor and an electric heater. The inlet temperature of a drier is preferably 100°C to 400°C, and more preferably 150°C to 300°C. The outlet temperature of the drier is preferably 100°C to 180°C, and more preferably 120°C to 170°C.

(Calcination Step)

The dried particle obtained as described above is calcined in air, and a calcined particle is obtained. The calcination is performed by use of a common tunnel or rotary kiln. The calcination temperature is preferably within a range of 500 to 750°C, and more preferably a range of 500 to 680°C. The calcination time may appropriately be adjusted depending on the calcination temperature, and is preferably within a range of 1 to 20 hours.

The shape of the calcined particle is not particularly limited, but is preferably spherical. The median diameter of the calcined particle is not particularly limited, but is preferably 10 to 180 $\mu$m. The median diameter of the calcined particle can be obtained by adopting water as a dispersion medium, placing 0.6 g of the calcined particle in 250 mL of water, performing an ultrasonic dispersion treatment for one minute, and then performing measurement under a condition of a relative refractive index of 1.40 with a laser diffraction/scattering particle size distribution measurement apparatus LA-300 manufactured by Horiba Ltd.

<Method for Producing Unsaturated Nitrile>

The catalyst for gas-phase catalytic ammoxidation reaction of the present embodiment can be used for the gas-phase catalytic ammoxidation reaction according to the first aspect or the gas-phase catalytic ammoxidation reaction according to the second aspect, thereby producing an unsaturated nitrile. In particular, acrylonitrile is preferably produced by use of the catalyst for gas-phase catalytic ammoxidation reaction of the present embodiment.

Hereinafter, the detail of the method for producing an unsaturated nitrile according to the present embodiment will be described with respect to each of the aspects.

[First Aspect]

In the present embodiment, an unsaturated nitrile can be produced by gas-phase catalytic ammoxidation reaction of a saturated hydrocarbon such as propane or isobutane in the presence of the catalyst for gas-phase catalytic ammoxidation reaction of the present embodiment.

(Starting Material)

Propane or isobutane, and ammonia, which are starting materials, do not necessarily have to be of high purity, and industrial grade gases such as propane containing 3 vol% or less of impurities such as ethane, ethylene, and n-butane, and ammonia containing 3 vol% or less of impurities such as water can be used. An oxygen-containing gas is not particularly limited, and for example, air, oxygen-enriched air, pure oxygen, a gas obtained by diluting any of these by an inert gas such as helium, argon, carbon dioxide, or nitrogen, or by steam can also be provided for the reaction. Among these, in a case where the oxygen-containing gas is used in an industrial scale, air is preferably used from the viewpoint of simplicity.

(Reaction Condition)

The gas-phase catalytic ammoxidation reaction of propane or isobutane can be performed under, for example, but not particularly limited to, the following conditions. The molar ratio of oxygen to be supplied for the reaction to propane or isobutane is preferably 0.1 to 6, and more preferably 0.5 to 4. The molar ratio of ammonia to be supplied for the reaction to propane or isobutane is preferably 0.3 to 1.5, and more preferably 0.7 to 1.2.

The reaction temperature is preferably 350 to 500°C, and more preferably 380 to 470°C. The reaction pressure is preferably $5 \times 10^4$ to $5 \times 10^5$ Pa, and more preferably $1 \times 10^5$ to $3 \times 10^5$ Pa. The contact time is preferably 0.1 to 10 sec·g/cm$^3$, and more preferably 0.5 to 5 sec·g/cm$^3$. By setting the conditions for the gas-phase catalytic ammoxidation reaction to the above-described ranges, there is a tendency that production of by-products is more suppressed and the yield of an unsaturated nitrile can be more improved.

In the present embodiment, the contact time is defined by the following equation.

$$\text{Contact time (sec·g/cm}^3) = (W/F) \times 273/(273 + T)$$

wherein W, F, and T are defined as follows.

W = amount (g) of catalyst filled
F = flow rate (Ncm$^3$/sec) of starting material mixed gas in standard state (0°C, $1.013 \times 10^5$ Pa)
T = reaction temperature (°C)

As a reaction system in the gas-phase catalytic ammoxidation reaction, a conventional system, such as a fixed bed, a fluidized bed, and a movable bed, can be adopted. Among these, a fluidized bed reactor with which removal of the heat of reaction is easy is preferable. The gas-phase catalytic ammoxidation reaction may be a single flow type or a recycle type.

[Second Aspect]

In the present embodiment, an unsaturated nitrile can be produced by reaction of an unsaturated hydrocarbon such as propylene, oxygen, and ammonia in the presence of the catalyst for gas-phase catalytic ammoxidation reaction of the present embodiment. Such production is preferably performed by fluidized bed ammoxidation reaction. An unsaturated nitrile can also be produced with the same reactor as a fluid bed reactor usable for the production of the catalyst for gas-phase catalytic ammoxidation reaction. In the present embodiment, acrylonitrile and hydrogen cyanide are preferably produced.

In the present embodiment, acrylonitrile may be produced in, for example, a fluid bed reactor usually used.

Propylene and ammonia, which are starting materials, do not necessarily have to be of high purity, and industrial grade propylene and ammonia can be used. While air is preferably used as a molecular oxygen source, a gas can also be used which is increased in oxygen concentration by mixing of oxygen with air.

In the present embodiment, when the oxygen source in production of acrylonitrile is air, the composition (molar ratio of ammonia and air to propylene; propylene/ammonia/air) of a starting material gas is preferably within a range of 1/(0.8 to 2.5)/(7.0 to 12.0), and more preferably a range of 1/(0.9 to 1.3)/(8.0 to 11.0) .

In the present embodiment, the reaction temperature in production of acrylonitrile is preferably within a range of 300 to 500°C, and more preferably a range of 400 to 480°C. The reaction pressure is preferably within a range of ordinary pressure to 0.3 MPa. The contact time between the starting material gas and the catalyst is preferably 0.5 to 20 (sec·g/cc), and more preferably 1 to 10 (sec·g/cc).

Examples

Hereinafter, the present embodiment will be described in more detail giving specific Examples and Comparative Examples, but the present embodiment is not limited at all by the following Examples and Comparative Examples within a range not exceeding the gist of the present embodiment.

[Example 1]

A catalyst particle where 44.4% by mass of a metal oxide having a metal component composition represented by $Mo_1V_{0.23}Sb_{0.24}Nb_{0.14}W_{0.03}Ce_{0.006}O_n$ was carried by 55.6% by mass of a carrier made of silica was produced according to the following procedure.

(Preparation of Niobium Starting Material Liquid)

A niobium starting material liquid was prepared by the following method. First of all, in a mixing tank, 77.8 kg of water was added, and the water was then heated to 45°C. Thereafter, 72.2 kg of oxalic acid dihydrate $[H_2C_2O_4·2H_2O]$ was loaded under stirring, and 20.0 kg of niobic acid containing 76.0% by mass of $Nb_2O_5$ was subsequently loaded to mix the two in the water. An aqueous mixed liquid obtained by subjecting this liquid to stirring under heating at 70°C for 8 hours was left to stand and cooled with ice, and a solid was then filtered and separated by suction filtration to obtain a uniform niobium starting material liquid. The obtained niobium starting material liquid was used as a niobium starting material liquid also in each catalyst production of the subsequent Examples and Comparative Examples.

The oxalic acid and niobium concentrations in the niobium starting material liquid were calculated as described below. The Nb concentration in the niobium starting material liquid was calculated from the weight of solid $Nb_2O_5$ obtained in such a way that 10 g of the niobium starting material liquid was precisely weighed in a crucible, dried at 120°C for 2 hours, and then subjected to a heating treatment at 600°C for 2 hours to find that the Nb concentration was 0.59 mol/kg. In a 300-mL glass beaker, 3 g of niobium starting material liquid was precisely weighed, 20 mL of hot water of about 80°C was added, and 10 mL of 1:1 sulfuric acid was subsequently added. A mixed liquid obtained in this way was titrated under stirring using 1/4N $KMnO_4$ while the liquid temperature was being kept at 70°C in a water bath. The point where a faint, pale pink color due to $KMnO_4$ was continued about 30 seconds or longer was defined as the end point. The oxalic acid concentration was calculated from the titrated amount according to the following formula to find that the oxalic acid concentration was found to be 1.61 mol/kg.

$$2KMnO_4 + 3H_2SO_4 + 5H_2C_2O_4 \rightarrow K_2SO_4 + 2MnSO_4 + 10CO_2 + 8H_2O$$

(Preparation of Precursor Slurry)

To 43.62 kg of water, 8.21 kg of ammonium heptamolybdate $[(NH_4)_6Mo_7O_{24}·4H_2O]$, 1.24 kg of ammonium metavanadate $[NH_4VO_3]$, 1.62 kg of diantimony trioxide $[Sb_2O_3]$, and 0.12 kg of cerium nitrate were added, and a resultant mixture was heated at 95°C for 1 hour under stirring to prepare an aqueous mixed liquid in liquid form.

After the aqueous mixed liquid was cooled to 70°C, 24.66 kg of silica sol containing 34.0% by mass of $SiO_2$ was added, furthermore 3.23 kg of a hydrogen peroxide solution containing 35.5% by mass of $H_2O_2$ was added, a resultant mixture was cooled to 55°C, and then 10.96 g of the niobium starting material liquid, 47.15 kg of a dispersion liquid containing 10.0% by mass of powder silica (dry silica), and 0.64 kg of an ammonium metatungstate liquid containing 50.2% by weight of tungsten oxide were added in sequence. The obtained mixed liquid was heated to 65°C, subjected to reaction for 2 hours under stirring, and then cooled to 50°C, and an aqueous solution where 0.086 kg of ammonium fluoride ($NH_4F$) was dissolved in 10 kg of water was then added and stirred for 0.5 hours, thereby obtaining a precursor slurry.

(Preparation of Dried Powder)

Next, the precursor slurry obtained as described above was supplied to a centrifugal spray drier and dried to obtain a microspherical dried powder having an average particle diameter of 50 $\mu$m. The inlet temperature and the outlet temperature of the drier were respectively 210°C and 120°C.

(Calcination of Dried Powder)

In a SUS calcination pipe with an inner diameter of 3 inches (76 mm), a length of 300 mm, and a thickness of 3 mm, 500 g of the dried powder obtained as described above was filled and was subjected to preliminary calcination and final calcination under a nitrogen flow of 5.0 NL/min while the calcination pipe was being rotated with the longitudinal direction thereof being as an axis. In the preliminary calcination, the temperature was raised from room temperature to 340°C at a temperature raising rate of 0.75°C/min and calcination was made at 340°C for 1 hour. Subsequently, in the final calcination, the temperature was raised from 340°C to 680°C at a temperature raising rate of 3°C/min, retained at 680°C for 1 hour and then decreased to 350°C at a temperature decreasing rate of 1°C/min, and thus a calcined product was obtained.

(Removal of Protrusion)

A protrusion present in a calcined product particle surface was removed by the following method. Into a vertical tube (inner diameter 41.6 mm, length 70 cm) provided with a holed disc with three holes having a diameter of 1/64 inches at the bottom portion and having a paper filter installed at the upper portion was placed 50 g of the calcined product. The length of the air flow on that occasion in a direction to which the air flowed was 52 mm, and the average linear velocity of the air flow was 310 m/s. A protrusion did not exist in the obtained catalyst 24 hours later.

(Confirmation of Crystal Phase)

The catalyst obtained as described above was subjected to XRD measurement, and as a result, a crystal phase constituted by MoVSbNb was confirmed.

[Example 2]

A catalyst particle where 44.4% by mass of a metal oxide having a metal component composition represented by $Mo_1V_{0.23}Sb_{0.24}Nb_{0.14}W_{0.03}Ce_{0.006}O_n$ was carried by 55.6% by mass of a carrier made of silica was produced according to the following procedure.
A catalyst was obtained by the same operation as in Example 1 except that the amount of compounding of the silica sol was 21.15 kg, the amount of compounding of the powder silica dispersion liquid was 58.83 kg and the amount of compounding of the ammonium fluoride was 0.017 kg in preparation of a precursor slurry, and furthermore the temperature was raised to 685°C in the final calcination.
The catalyst obtained as described above was subjected to XRD measurement in the same manner as in Example 1, and as a result, a crystal phase constituted by MoVSbNb was confirmed.

[Example 3]

A catalyst was obtained by the same operation as in Example 1 except that the temperature was raised to 640°C in the final calcination.
The catalyst obtained as described above was subjected to XRD measurement in the same manner as in Example 1, and as a result, a crystal phase constituted by MoVSbNb was confirmed.

[Comparative Example 1]

A catalyst was obtained by the same operation as in Example 1 except that no ammonium fluoride was compounded in preparation of a precursor slurry.
The catalyst obtained as described above was subjected to XRD measurement in the same manner as in Example 1, and as a result, a crystal phase constituted by MoVSbNb was confirmed.

[Comparative Example 2]

A catalyst particle where 44.4% by mass of a metal oxide having a metal component composition represented by $Mo_1V_{0.23}Sb_{0.24}Nb_{0.14}W_{0.03}Ce_{0.006}O_n$ was carried by 55.6% by mass of a carrier made of silica was produced according to the following procedure.

A catalyst was obtained by the same operation as in Example 1 except that the amount of compounding of the silica sol was 21.15 kg, the amount of compounding of the powder silica dispersion liquid was 58.83 kg and the amount of compounding of the ammonium fluoride was 0.001 kg in preparation of a precursor slurry, and furthermore the temperature was raised to 685°C in the final calcination.

The catalyst obtained as described above was subjected to XRD measurement in the same manner as in Example 1, and as a result, a crystal phase constituted by MoVSbNb was confirmed.

[Comparative Example 3]

A catalyst was obtained by the same operation as in Example 1 except that the amount of compounding of the ammonium fluoride was 8.60 kg in preparation of a precursor slurry.

The catalyst obtained as described above was subjected to XRD measurement in the same manner as in Example 1, and as a result, a crystal constituted by MoVSbNb was not confirmed.

[Example 4]

A catalyst particle where 60% by mass of a metal oxide having a metal component composition represented by $Mo_{12.00}Bi_{0.37}Fe_{1.42}Co_{4.47}Ni_{3.30}Ce_{0.91}Rb_{0.14}$ was carried by 40% by mass of a carrier made of silica was produced according to the following procedure.

In a 650-L container provided with a stirring apparatus, 71.90 kg of ammonium paramolybdate $[(NH_4)_6Mo_7O_{24}·4H_2O]$ dissolved in 128.34 kg of water was added to a mixed liquid where 3.75 kg of oxalic acid dehydrate dissolved in 43.13 kg of water and an aqueous solution of 0.60 kg of ammonium fluoride $(NH_4F)$ dissolved in 10 kg of water were added to 176.47 kg of silica sol containing 30% by mass of $SiO_2$, under stirring, thereby obtaining a first solution containing molybdenum and silica, and the liquid temperature was adjusted to 45°C.

Next, 6.14 kg of bismuth nitrate $[Bi(NO_3)_3·5H_2O]$, 19.39 kg of iron nitrate $[Fe(NO_3)_3·9H_2O]$, 44.56 kg of cobalt nitrate $[Co(NO_3)_2·6H_2O]$, 32.90 kg of nickel nitrate $[Ni(NO_3)_2·6H_2O]$, 13.36 kg of cerium nitrate $[Ce(NO_3)_3·6H_2O]$, and 0.70 kg of rubidium nitrate $[RbNO_3]$ were dissolved in 59.02 kg of 16.6% by mass of nitric acid, thereby obtaining a second solution, and the liquid temperature was adjusted to 40°C.

The second solution was mixed with the first solution, thereby obtaining a precursor slurry.

The obtained precursor slurry was dried with a rotary disc type spray drier with an inner diameter of the drier of 5400 mm. The air temperature at the drier inlet was here 220°C. The number of rotations of the disc was set to 7000 rpm.

A resultant dried particle was calcined at 600°C for 2 hours, thereby obtaining a catalyst.

The catalyst obtained as described above was subjected to XRD measurement in the same manner as in Example 1, and as a result, a crystal phase constituted by MoBiFe was confirmed.

[Example 5]

A catalyst was obtained in the same manner as in Example 4 except that 1.04 kg of the ammonium fluoride was used.

The catalyst obtained as described above was subjected to XRD measurement in the same manner as in Example 1, and as a result, a crystal phase constituted by MoBiFe was confirmed.

[Comparative Example 4]

A catalyst was obtained in the same manner as in Example 4 except that no ammonium fluoride was used.

The catalyst obtained as described above was subjected to XRD measurement in the same manner as in Example 1, and as a result, a crystal phase constituted by MoBiFe was confirmed.

[Example 6]

A catalyst particle where 40% by mass of a metal oxide having a metal component composition represented by $Mo_1V_{0.23}Sb_{0.24}Nb_{0.14}W_{0.03}Ce_{0.006}O_n$ was carried by 60% by mass of a carrier made of silica was produced according to the following procedure.

A catalyst was obtained by the same operation as in Comparative Example 1 except that the amount of compounding of the silica sol was 23.11 kg and the amount of compounding of the powder silica dispersion liquid was 78.59 kg in preparation of a precursor slurry, and furthermore the temperature was raised to 695°C and retained for 3 hours in the final calcination.

The catalyst obtained as described above was subjected to XRD measurement in the same manner as in Example 1, and as a result, a crystal phase constituted by MoVSbNb was confirmed.

[Example 7]

A catalyst particle where 40% by mass of a metal oxide having a metal component composition represented by $Mo_1V_{0.23}Sb_{0.24}Nb_{0.14}W_{0.03}Ce_{0.006}O_n$ was carried by 60% by mass of a carrier made of silica was produced according to the following procedure.

A catalyst was obtained by the same operation as in Comparative Example 1 except that the amount of compounding of the silica sol was 23.11 kg and the amount of compounding of the powder silica dispersion liquid was 78.59 kg in preparation of a precursor slurry, and furthermore the temperature was raised to 695°C and retained for 4 hours in the final calcination.

The catalyst obtained as described above was subjected to XRD measurement in the same manner as in Example 1, and as a result, a crystal phase constituted by MoVSbNb was confirmed.

[Example 8]

A catalyst particle where 40% by mass of a metal oxide having a metal component composition represented by $Mo_1V_{0.23}Sb_{0.24}Nb_{0.14}W_{0.03}Ce_{0.006}O_n$ was carried by 60% by mass of a carrier made of silica was produced according to the following procedure.

A catalyst was obtained by the same operation as in Comparative Example 1 except that the amount of compounding of the silica sol was 18.49 kg and the amount of compounding of the powder silica dispersion liquid was 94.31 kg in preparation of a precursor slurry, and furthermore the temperature was raised to 695°C and retained for 3 hours in the final calcination.

The catalyst obtained as described above was subjected to XRD measurement in the same manner as in Example 1, and as a result, a crystal phase constituted by MoVSbNb was confirmed.

[Comparative Example 5]

A catalyst particle where 40% by mass of a metal oxide having a metal component composition represented by $Mo_1V_{0.23}Sb_{0.24}Nb_{0.14}W_{0.03}Ce_{0.006}O_n$ was carried by 60% by mass of a carrier made of silica was produced according to the following procedure.

A catalyst was obtained by the same operation as in Comparative Example 1 except that the amount of compounding of the silica sol was 23.11 kg and the amount of compounding of the powder silica dispersion liquid was 78.59 kg in preparation of a precursor slurry, and furthermore the temperature was raised to 695°C and retained for 1 hours in the final calcination.

The catalyst obtained as described above was subjected to XRD measurement in the same manner as in Example 1, and as a result, a crystal phase constituted by MoVSbNb was confirmed.

[Comparative Example 6]

A catalyst particle where 40% by mass of a metal oxide having a metal component composition represented by $Mo_1V_{0.23}Sb_{0.24}Nb_{0.14}W_{0.03}Ce_{0.006}O_n$ was carried by 60% by mass of a carrier made of silica was produced according to the following procedure.

A catalyst was obtained by the same operation as in Comparative Example 1 except that the amount of compounding of the silica sol was 23.11 kg and the amount of compounding of the powder silica dispersion liquid was 78.59 kg in preparation of a precursor slurry, and furthermore the temperature was raised to 680°C and retained for 3 hours in the final calcination.

The catalyst obtained as described above was subjected to XRD measurement in the same manner as in Example 1, and as a result, a crystal phase constituted by MoVSbNb was confirmed.

[Example 9]

A catalyst particle where 30% by mass of a metal oxide having a metal component composition represented

by $Mo_1V_{0.23}Sb_{0.24}Nb_{0.14}W_{0.03}Ce_{0.006}O_n$ was carried by 70% by mass of a carrier made of silica was produced according to the following procedure.

A catalyst was obtained by the same operation as in Comparative Example 1 except that a precursor slurry was prepared as follows and furthermore the temperature was raised to 685°C and retained for 2 hours in the final calcination.

(Preparation of Precursor Slurry)

To 32.7 kg of water, 6.16 kg of ammonium heptamolybdate $[(NH_4)_6Mo_7O_{24} \cdot 4H_2O]$, 0.93 kg of ammonium metavanadate $[NH_4VO_3]$, 1.21 kg of diantimony trioxide $[Sb_2O_3]$, and 0.09 kg of cerium nitrate were added, and a resultant mixture was heated at 95°C for 1 hour under stirring to prepare an aqueous mixed liquid in liquid form.

After the aqueous mixed liquid was cooled to 70°C, 7.71 kg of silica sol containing 34.0% by mass of $SiO_2$ and 100.50 kg of a dispersion liquid containing 10.0% by mass of powder silica were added, furthermore 3.23 kg of a hydrogen peroxide solution containing 35.5% by mass of $H_2O_2$ was added, a resultant mixture was cooled to 55°C, and then 6.38 g of the niobium starting material liquid, 52.39 kg of a dispersion liquid containing 10.0% by mass of powder silica, and 0.48 kg of an ammonium metatungstate liquid containing 50.2% by weight of tungsten oxide were added in sequence. The obtained mixed liquid was heated to 65°C, subjected to reaction for 2 hours under stirring, and then cooled to 50°C and thereafter stirred for 0.5 hours, thereby obtaining a precursor slurry.

The catalyst obtained as described above was subjected to XRD measurement in the same manner as in Example 1, and as a result, a crystal phase constituted by MoVSbNb was confirmed.

[Example 10]

A catalyst particle where 30% by mass of a metal oxide having a metal component composition represented by $Mo_1V_{0.23}Sb_{0.24}Nb_{0.14}W_{0.03}Ce_{0.006}O_n$ was carried by 70% by mass of a carrier made of silica was produced according to the following procedure.

A catalyst was obtained by the same operation as in Comparative Example 1 except that a precursor slurry was prepared as follows and furthermore the temperature was raised to 685°C and retained for 2 hours in the final calcination.

(Preparation of Precursor Slurry)

To 32.7 kg of water, 6.16 kg of ammonium heptamolybdate $[(NH_4)_6Mo_7O_{24} \cdot 4H_2O]$, 0.93 kg of ammonium metavanadate $[NH_4VO_3]$, 1.21 kg of diantimony trioxide $[Sb_2O_3]$, and 0.09 kg of cerium nitrate were added, and a resultant mixture was heated at 95°C for 1 hour under stirring to prepare an aqueous mixed liquid in liquid form.

After the aqueous mixed liquid was cooled to 70°C, 131.0 kg of a dispersion liquid containing 10.0% by mass of powder silica was added, furthermore 3.23 kg of a hydrogen peroxide solution containing 35.5% by mass of $H_2O_2$ was added, a resultant mixture was cooled to 55°C, and then 6.38 g of the niobium starting material liquid, 52.39 kg of a dispersion liquid containing 10.0% by mass of powder silica, and 0.48 kg of an ammonium metatungstate liquid containing 50.2% by weight of tungsten oxide were added in sequence. The obtained mixed liquid was heated to 65°C, subjected to reaction for 2 hours under stirring, and then cooled to 50°C and thereafter stirred for 0.5 hours, thereby obtaining a precursor slurry.

The catalyst obtained as described above was subjected to XRD measurement in the same manner as in Example 1, and as a result, a crystal phase constituted by MoVSbNb was confirmed.

[Comparative Example 7]

A catalyst particle where 30% by mass of a metal oxide having a metal component composition represented by $Mo_1V_{0.23}Sb_{0.24}Nb_{0.14}W_{0.03}Ce_{0.006}O_n$ was carried by 70% by mass of a carrier made of silica was produced according to the following procedure.

A catalyst was obtained by the same operation as in Comparative Example 1 except that a precursor slurry was prepared as follows and furthermore the temperature was raised to 685°C and retained for 2 hours in the final calcination.

(Preparation of Precursor Slurry)

To 32.7 kg of water, 6.16 kg of ammonium heptamolybdate $[(NH_4)_6Mo_7O_{24} \cdot 4H_2O]$, 0.93 kg of ammonium metavanadate $[NH_4VO_3]$, 1.21 kg of diantimony trioxide $[Sb_2O_3]$, and 0.09 kg of cerium nitrate were added, and a resultant mixture was heated at 95°C for 1 hour under stirring to prepare an aqueous mixed liquid in liquid form.

After the aqueous mixed liquid was cooled to 70°C, 7.71 kg of silica sol containing 34.0% by mass of $SiO_2$ was added, furthermore 3.23 kg of a hydrogen peroxide solution containing 35.5% by mass of $H_2O_2$ was added, a resultant mixture was cooled to 55°C, and then 6.38 g of the niobium starting material liquid, 152.89 kg of a dispersion liquid containing 10.0% by mass of powder silica, and 0.48 kg of an ammonium metatungstate liquid containing 50.2% by weight of tungsten oxide were added in sequence. The obtained mixed liquid was heated to 65°C, subjected to reaction for 2 hours under stirring, and then cooled to 50°C and thereafter stirred for 0.5 hours, thereby obtaining a precursor slurry.

The catalyst obtained as described above was subjected to XRD measurement in the same manner as in Example 1, and as a result, a crystal phase constituted by MoVSbNb was confirmed.

[Comparative Example 8]

A catalyst particle where 30% by mass of a metal oxide having a metal component composition represented by $Mo_1V_{0.23}Sb_{0.24}Nb_{0.14}W_{0.03}Ce_{0.006}O_n$ was carried by 70% by mass of a carrier made of silica was produced according to the following procedure.

A catalyst was obtained by the same operation as in Comparative Example 1 except that a precursor slurry was prepared as follows and furthermore the temperature was raised to 685°C and retained for 2 hours in the final calcination.

(Preparation of Precursor Slurry)

To 32.7 kg of water, 6.16 kg of ammonium heptamolybdate $[(NH_4)_6Mo_7O_{24}\cdot 4H_2O]$, 0.93 kg of ammonium metavanadate $[NH_4VO_3]$, 1.21 kg of diantimony trioxide $[Sb_2O_3]$, and 0.09 kg of cerium nitrate were added, and a resultant mixture was heated at 95°C for 1 hour under stirring to prepare an aqueous mixed liquid in liquid form.

After the aqueous mixed liquid was cooled to 70°C, 3.23 kg of a hydrogen peroxide solution containing 35.5% by mass of $H_2O_2$ was added, a resultant mixture was cooled to 55°C, and then 6.38 g of the niobium starting material liquid, 183.38 kg of a dispersion liquid containing 10.0% by mass of powder silica, and 0.48 kg of an ammonium metatungstate liquid containing 50.2% by weight of tungsten oxide were added in sequence. The obtained mixed liquid was heated to 65°C, subjected to reaction for 2 hours under stirring, and then cooled to 50°C and thereafter stirred for 0.5 hours, thereby obtaining a precursor slurry.

The catalyst obtained as described above was subjected to XRD measurement in the same manner as in Example 1, and as a result, a crystal phase constituted by MoVSbNb was confirmed.

Various physical property and performance evaluations in Examples and Comparative Examples were performed by the following methods.

<F/Mo Ratio in Catalyst>

The composition F/Mo in the catalyst was quantitatively determined by a fundamental-parameter (FP) method with X-ray fluorescence analysis (trade name "RIX 1000" manufactured by Rigaku Corporation, Cr tube, tube voltage 50 kV, tube current 50 mA). Specifically, the obtained catalyst was pulverized/mixed for two hours using a uniaxial type agate automatic mortar (manufactured by NITTO KAGAKU CO., LTD.), and press-molded with a uniaxial press into a vinyl chloride ring (manufactured by Rigaku Corporation). Measurement with semi-quantitative analysis was performed on the obtained pellet by a fundamental-parameter (FP) method in which a content ratio is determined from the sensitivity library registered on the software in advance using wavelength dispersive X-ray fluorescence analysis (trade name "RIX 1000" manufactured by Rigaku Corporation, Cr tube, tube voltage 50 kV, tube current 50 mA). The results obtained are shown in "F/Mo (catalyst) " in the Table.

<Water Vapor Adsorption Measurement>

Water vapor adsorption measurement was performed as described below.

Into a glass sample tube was placed about 0.5 g of a sample before drying, and subjected to heating and degassing in vacuum with a sample pre-treatment apparatus ("VacPrep061" manufactured by Micromeritics Instrument Corp.) under conditions of 250°C and 0.001 mmHg or less for about 18 hours. The measurement value after heating and degassing in vacuum was adopted as the dry weight of the sample in order to obtain the amount of adsorption per unit weight. Water vapor adsorption measurement was performed at a measurement temperature of 25°C with water vapor used as an adsorption gas by use of a multi-specimen high-performance specific surface area/pore distribution measurement apparatus "3Flex" manufactured by Micromeritics Instrument Corp. The interaction coefficient C (BET parameter C as a name according to JIS) was calculated from the intercept and slope values by applying values on adsorption isotherm at 10 or more points evenly dividing a range of relative pressure of 0.05 or more and 0.3 or less, to the following BET expression.

$$1/V[(p_0/p)-1] = (C-1)/V_mC \times (p/p_0) + 1/(V_mC)$$

Here, V represents the amount of adsorption of water vapor at each measurement point, $p/p_o$ represents the relative pressure at each measurement, $V_m$ represents the amount of monomolecular adsorption, and C represents the interaction coefficient.

<Measurement of Specific Surface Area>

The specific surface area was determined by a BET 1-point method with Gemini 2360 manufactured by Micromeritics Instrument Corp., after a pre-treatment by heating 0.5 g of the catalyst in a glass cell at 300°C for 15 minutes under a nitrogen gas flow.

<Reaction Evaluation of Ammoxidation Reaction of Propane>

Propane was provided for gas-phase catalytic ammoxidation reaction by the following method using the catalyst obtained as described above. In a VYCOR glass fluidized bed type reaction pipe with an inner diameter of 25 mm, 40 g of the catalyst was filled, and a mixed gas having a molar ratio of propane:ammonia:oxygen:helium = 1:1.1:2.9:11.6 was supplied at a contact time of 3.0 (sec·g/cm$^3$) under a reaction temperature of 445°C and a reaction pressure of 40 kPa.
The yield of acrylonitrile was determined as follows. After a calibration curve was acquired in advance by analyzing a gas of acrylonitrile having a known concentration by gas chromatography (GC: product name "GC 2014" manufactured by SHIMADZU CORPORATION), a gas produced by the ammoxidation reaction was injected quantitatively in GC, and the number of moles of acrylonitrile produced was measured. The yield of acrylonitrile was determined from the number of moles of acrylonitrile measured according to the following equation.

$$\text{Yield of acrylonitrile (\%)} = (\text{number of moles of acrylonitrile produced})/(\text{number of moles of propane supplied}) \times 100$$

The reaction was continuously performed with this catalyst, and the reaction yield of acrylonitrile (AN) measured 10 days after starting the reaction is shown in Table 1.

<Reaction Evaluation of Ammoxidation Reaction of Propylene>

The catalysts obtained in Examples and Comparative Examples were each used to produce acrylonitrile and hydrogen cyanide by ammoxidation reaction of propylene. A reaction pipe here used was a PYREX (registered trade mark) glass pipe with an inner diameter of 25 mm, in which sixteen 10-mech wire nets were embedded at an interval of 1 cm.
The amount of the catalyst, the reaction temperature and the reaction pressure were respectively set to 50 cc, 430°C and 0.17 MPa, and a mixed gas of propylene/ammonia/air was supplied at a total gas flow rate of 250 to 450 cc/sec (in terms of NTP) to thereby perform the reaction. As long as the content of propylene in the mixed gas was 9% by volume and the molar ratio of propylene/ammonia/air was 1/(0.7 to 2.5)/(8.0 to 13.5), the flow rate of ammonia was appropriately changed so that the sulfuric acid unit requirement defined by the following expression was 20 ± 2 kg/T-AN and the flow rate of air was appropriately changed so that the oxygen concentration of the reactor outlet gas was 0.2 ± 0.02% by volume. The flow rate of the entire mixed gas was changed and thus the contact time defined by the following expression was changed and the propylene conversion defined by the following expression was set to 99.3 ± 0.2%.
The yield of acrylonitrile and the yield of hydrogen cyanide, generated by the reaction, were values defined by the following expressions. The amount of substance of a product was defined based on the number of carbon atoms of propylene as a starting material. The yield of acrylonitrile measured is shown in Table 1.

$$\text{Sulfuric acid unit requirement (kg/T-AN)} = \frac{\text{weight of sulfuric acid necessary for neutralization of unreacted ammonia(kg)}}{\text{weight of production of acrylonitrile(T)}}$$

$$\text{Contact time (sec.)} = \frac{\text{amount of catalyst(cc)}}{\text{flow rate of mixed gas(cc-NTP/sec.)}} \times \frac{273}{273 + \text{reaction temperature (°C)}}$$
$$\times \frac{\text{reaction pressure (MPa)}}{0.10}$$

$$\text{Conversion of propylene(\%)} = \frac{\text{propylene consumed (mol)}}{\text{propylene supplied (mol)}} \times 100$$

$$\text{Yield of acrylonitrile(\%)} = \frac{\text{acrylonitrile produced (mol)}}{\text{propylene supplied (mol)}} \times 100$$

$$\text{Yield of hydrogen cyanide(\%)} = \frac{\text{hydrogen cyanide produced (mol)}}{\text{propylene supplied (mol)}} \times 100$$

<Combustion Rate of Ammonia>

The combustion rate of ammonia was determined from the number of moles of nitrogen measured in each of reaction evaluation of ammoxidation reaction of propane and reaction evaluation of ammoxidation reaction of propylene, according to the following expression. When the combustion rate of ammonia was within a range of 10.0 to 22.0%, it was determined that ammonia and hydrocarbon as starting materials were efficiently usable.

```
Combustion rate of ammonia (%) = (number of moles of

nitrogen produced × 2)/(number of moles of propane

supplied) × 100
```

[Table 1]

| | Application (starting material) | F/Mo (Loading) | Calcination temperature (°C) | Calcination time (h) | Powder silica (% by mass) | Proportion of powder silica in silica starting material(% by mass) | Specific surface area (m2/g) | F/Mo (Catalyst) | Interaction coefficient C | Yield of AN (%) | Combustion rate of NH3 (%) |
|---|---|---|---|---|---|---|---|---|---|---|---|
| Example 1 | Saturated hydrocarbon | 0.05 | 680 | 1 | 20 | 36 | 9.1 | 0.015 | 3.9 | 56.8 | 15.5 |
| Example 2 | Saturated hydrocarbon | 0.01 | 685 | 1 | 25 | 45 | 8.7 | 0.002 | 3.7 | 57.0 | 16.0 |
| Example 3 | Saturated hydrocarbon | 0.05 | 640 | 1 | 20 | 36 | 10.8 | 0.021 | 5.1 | 56.5 | 150 |
| Comparative Example 1 | Saturated hydrocarbon | 0 | 680 | 1 | 20 | 36 | 15.1 | - | 40.5 | 52.5 | 24 |
| Comparative Example 2 | Saturated hydrocarbon | 0.0005 | 685 | 1 | 25 | 45 | 14.5 | 0.00001 | 35 | 53.1 | 23.5 |
| Comparative Example 3 | Saturated hydrocarbon | 5 | 680 | 1 | 20 | 36 | 6.1 | 22 | 15.1 | - | - |
| Example 4 | Unsaturated hydrocarbon | 0.04 | 600 | 2 | 0 | 0 | 32.8 | 0.018 | 18.5 | 84.5 | 21.9 |
| Example 5 | Unsaturated hydrocarbon | 0.07 | 600 | 2 | 0 | 0 | 32.3 | 0.035 | 17.4 | 84.4 | 17.7 |
| Comparative Example 4 | Unsaturated hydrocarbon | 0 | 600 | 2 | 0 | 0 | 29.3 | - | 45.1 | 84.1 | 28.0 |
| Example 6 | Saturated hydrocarbon | 0 | 695 | 3 | 30 | 50 | 14.2 | | 10.5 | 56.5 | 20.5 |
| Example 7 | Saturated hydrocarbon | 0 | 695 | 4 | 30 | 50 | 13 | | 9.1 | 56.9 | 19.5 |
| Example 8 | Saturated hydrocarbon | 0 | 695 | 3 | 36 | 60 | 14.9 | | 10 | 56.7 | 20 |
| Comparative Example 5 | Saturated hydrocarbon | 0 | 695 | 1 | 30 | 50 | 16 | | 45.7 | 52.3 | 25 |

(continued)

| Application (starting material) | F/Mo (Loading) | Calcination temperature (°C) | Calcination time (h) | Powder silica (% by mass) | Proportion of powder silica in silica starting material (% by mass) | Specific surface area (m2/g) | F/Mo (Catalyst) | Interaction coefficient C | Yield of AN (%) | Combustion rate of NH3 (%) |
|---|---|---|---|---|---|---|---|---|---|---|
| Comparative Example 6 | Saturated hydrocarbon | 0 | 680 | 3 | 30 | 50 | 19.5 | | 40 | 52.6 | 24.5 |
| Example 9 | Saturated hydrocarbon | 0 | 685 | 2 | 60 | 85 | 19 | | 15 | 56 | 21.5 |
| Example 10 | Saturated hydrocarbon | 0 | 685 | 2 | 70 | 100 | 19.5 | | 14.6 | 56.2 | 20 |
| Comparative Example 7 | Saturated hydrocarbon | 0 | 685 | 2 | 60 | 85 | 20.1 | | 35.3 | 50.1 | 25 |
| Comparative Example 8 | Saturated hydrocarbon | 0 | 685 | 2 | 70 | 100 | 21.5 | | 34.1 | 49.5 | 24.5 |

In Comparative Example 3, a crystal phase capable of sufficiently catalyzing ammoxidation reaction (crystal phase constituted by MoVSbNb) was not generated, and this was evaluated as failing to provide a catalyst for gas-phase catalytic ammoxidation reaction. This was considered to be due to an excess amount of addition of the fluorine compound.

[Measurement of Attrition strength]

The attrition strength of the catalyst was measured according to a method described in "Test Method for Synthetic Fluid Cracking Catalyst" (American Cyanamid Co. Ltd. 6/31-4 m-1/57).
The attrition strength was evaluated by the wear loss, and the wear loss was a value defined as follows.

$$\text{Wear loss (\%) = R/(S-Q)} \times 100$$

In the expression, Q represents the mass (g) of the catalyst worn and scattered externally for 0 to 5 hours, R represents the mass (g) of the catalyst worn and scattered externally for usual 5 to 20 hours, and S represents the mass (g) of the catalyst examined.
The attrition strength of each of the catalysts obtained in Example 1 and Comparative Example 1 was measured by the above-mentioned method, and as a result, that in Example 1 was 1% and that in Comparative Example 1 was 2.9%. While both the catalysts thus had sufficient strength, the catalyst obtained in Example 1 was evaluated as being more excellent in strength than the catalyst obtained in Comparative Example 1.
The catalysts obtained in Examples 2 to 10 were also evaluated as having sufficient strength.
The present application claims priority to Japanese Patent Application (Japanese Patent Application No. 2020-218492) filed on December 28, 2020, the content of which is herein incorporated as reference.

**Claims**

1.   A catalyst for gas-phase catalytic ammoxidation reaction, the catalyst comprising a metal oxide and a silica carrier carrying the metal oxide, wherein

the metal oxide comprises molybdenum, and
an interaction coefficient C between water vapor and the catalyst for gas-phase catalytic ammoxidation reaction, the interaction coefficient C being derived by subjecting the catalyst for gas-phase catalytic ammoxidation reaction to a water vapor adsorption measurement, is 1 or more and 30 or less.

2.   The catalyst for gas-phase catalytic ammoxidation reaction according to claim 1, wherein a mass proportion of the silica carrier is 30% by mass or more and 70% by mass or less in terms of $SiO_2$ based on a total amount of the catalyst for gas-phase catalytic ammoxidation reaction.

3.   The catalyst for gas-phase catalytic ammoxidation reaction according to claim 1 or 2, wherein the metal oxide is represented by the following formula (1):

$$Mo_1V_aSb_bNb_cW_dX_eO_n \cdots \qquad (1)$$

wherein X represents at least one selected from the group consisting of Te, Ce, Ti and Ta; a, b, c, and d satisfy relational expressions of $0.01{\leq}a{\leq}1$, $0.01{\leq}b{\leq}1$, $0.01{\leq}c{\leq}1$, $0{\leq}d{\leq}1$, and $0{\leq}e{\leq}1$; and n represents a number determined by valences of the other elements.

4.   The catalyst for gas-phase catalytic ammoxidation reaction according to claim 1 or 2, wherein the metal oxide is represented by the following formula (2):

$$Mo_{12}Bi_fFe_gX_hY_iZ_jO_m \cdots \qquad (2)$$

wherein X represents at least one element selected from the group consisting of nickel, cobalt, magnesium, calcium, zinc, strontium and barium; Y represents at least one element selected from the group consisting of cerium, chromium, lanthanum, neodymium, yttrium, praseodymium, samarium, aluminum, gallium and indium; Z represents at least one element selected from the group consisting of potassium, rubidium and cesium; f, g, h, i, j and m represent atomic ratios of the elements, and respectively satisfy $0.1{\leq}f{\leq}2.0$, $0.1{\leq}g{\leq}3.0$, $0.1{\leq}h{\leq}10.0$, $0.1{\leq}i{\leq}3.0$, and $0.01{\leq}j{\leq}2.0$;

and m represents a number of oxygen atoms necessary for satisfying valence requirements of the other elements present.

5. The catalyst for gas-phase catalytic ammoxidation reaction according to any one of claims 1 to 4, further comprising fluorine.

6. The catalyst for gas-phase catalytic ammoxidation reaction according to claim 5, wherein a molar ratio of fluorine to molybdenum, F/Mo, is 0.0001 or more and 1 or less.

7. A method for producing acrylonitrile wherein the catalyst for gas-phase catalytic ammoxidation reaction according to any one of claims 1 to 6 is used.

8. A method for producing a catalyst for gas-phase catalytic ammoxidation reaction, the catalyst comprising a metal oxide and a silica carrier carrying the metal oxide, the method comprising

a preparation step of preparing a precursor slurry comprising a starting material for the silica carrier and Mo;
a drying step of spray drying the precursor slurry to obtain a dried particle; and
a calcination step of calcining the dried particle, and
at least one operation selected from the group consisting of the following (I), (II) and (III) is performed:

(I) adding a fluorine compound in the preparation step and/or the drying step;
(II) keeping a state of a calcination temperature of 695°C or more under an inert gas atmosphere for 3 hours or more in the calcination step; and
(III) by using, as a starting material for the silica carrier, a silica particle having an interaction coefficient C with water vapor of 1 or more and 10 or less, the interaction coefficient C being derived by a water vapor adsorption measurement, adding 50% by mass or more of the silica particle based on a total mass M of the starting material for the silica carrier in the preparation step, wherein the preparation step comprises a first silica particle addition step of adding at least a portion of the starting material for the silica carrier to a mixture comprising Mo, a Nb addition step of adding Nb to the mixture, and a second silica particle addition step of adding at least a portion of a balance of the starting material for the silica carrier to the mixture at the same time as the Nb addition step or after the Nb addition step, and wherein a ratio of a mass M1 of the starting material for the silica carrier to the total mass M, M1/M, in the first silica particle addition step is 0.4 to 0.7.

9. The method for producing the catalyst for gas-phase catalytic ammoxidation reaction according to claim 8, wherein the metal oxide is represented by the following formula (1):

$$Mo_1V_aSb_bNb_cW_dX_eO_n \cdots \qquad (1)$$

wherein X represents at least one selected from the group consisting of Te, Ce, Ti and Ta; a, b, c, and d satisfy relational expressions of $0.01{\leq}a{\leq}1$, $0.01{\leq}b{\leq}1$, $0.01{\leq}c{\leq}1$, $0{\leq}d{\leq}1$, and $0{\leq}e{\leq}1$; and n represents a number determined by valences of the other elements.

10. The method for producing the catalyst for gas-phase catalytic ammoxidation reaction according to claim 8, wherein the metal oxide is represented by the following formula (2):

$$Mo_{12}Bi_fFe_gX_hY_iZ_jO_m \cdots \qquad (2)$$

wherein X represents at least one element selected from the group consisting of nickel, cobalt, magnesium, calcium, zinc, strontium and barium; Y represents at least one element selected from the group consisting of cerium, chromium, lanthanum, neodymium, yttrium, praseodymium, samarium, aluminum, gallium and indium; Z represents at least one element selected from the group consisting of potassium, rubidium and cesium; f, g, h, i, j and m represent atomic ratios of the elements, and respectively satisfy $0.1{\leq}f{\leq}2.0$, $0.1{\leq}g{\leq}3.0$, $0.1{\leq}h{\leq}10.0$, $0.1{\leq}i{\leq}3.0$, and $0.01{\leq}j{\leq}2.0$; and m represents a number of oxygen atoms necessary for satisfying valence requirements of the other elements present.

11. The method for producing the catalyst for gas-phase catalytic ammoxidation reaction according to any one of claims 8 to 10, wherein the fluorine compound is added so that a molar ratio of fluorine to molybdenum, F/Mo, in the precursor slurry is 0.001 or more and 1 or less.

12. The method for producing the catalyst for gas-phase catalytic ammoxidation reaction according to any one of claims 8 to 11, wherein the fluorine compound comprises ammonium fluoride.

# EP 4 268 952 A1

| | INTERNATIONAL SEARCH REPORT | International application No. |
|---|---|---|
| | | **PCT/JP2021/048409** |

**A. CLASSIFICATION OF SUBJECT MATTER**

*B01J 27/132*(2006.01)i; *B01J 27/138*(2006.01)i; *B01J 37/00*(2006.01)i; *B01J 37/08*(2006.01)i; *C07B 61/00*(2006.01)i;
*C07C 253/24*(2006.01)i; *C07C 253/26*(2006.01)i; *C07C 255/08*(2006.01)i
FI: B01J27/132 Z; B01J27/138 Z; B01J37/00 F; B01J37/08; C07B61/00 300; C07C253/24; C07C253/26; C07C255/08

According to International Patent Classification (IPC) or to both national classification and IPC

**B. FIELDS SEARCHED**

Minimum documentation searched (classification system followed by classification symbols)

B01J27/132; B01J27/138; B01J37/00; B01J37/08; C07B61/00; C07C253/24; C07C253/26; C07C255/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2012/090979 A1 (ASAHI KASEI CHEMICALS CORPORATION) 05 July 2012 (2012-07-05) claims 1-10, paragraphs [0061]-[0063], [0126]-[0128], [0180] | 1-3, 7-9 |
| A | | 4-6, 10-12 |
| A | JP 2012-501839 A (BASF SE) 26 January 2012 (2012-01-26) entire text | 1-12 |
| A | JP 2000-501371 A (STUDIENGESELLSCHAFT KOHLE MBH) 08 February 2000 (2000-02-08) entire text, all drawings | 1-12 |
| P, A | CN 112316975 A (KUNMING UNIVERSITY OF SCIENCE AND TECHNOLOGY) 05 February 2021 (2021-02-05) entire text, all drawings | 1-12 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 February 2022** | **22 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2021/048409**

| Patent document cited in search report | | | Publication date (day/month/year) | Patent family member(s) | | | Publication date (day/month/year) |
|---|---|---|---|---|---|---|---|
| WO | 2012/090979 | A1 | 05 July 2012 | US | 2013/0253217 | A1 | |
| | | | | claims 1-22, paragraphs [0083]-[0087], [0160]-[0162], [0225] | | | |
| | | | | EP | 2659965 | A1 | |
| | | | | CN | 103269790 | A | |
| | | | | KR | 10-2013-0086376 | A | |
| JP | 2012-501839 | A | 26 January 2012 | US | 2010/0069660 | A1 | |
| | | | | entire text | | | |
| | | | | WO | 2010/028991 | A1 | |
| | | | | EP | 2323760 | A1 | |
| | | | | KR | 10-2011-0063533 | A | |
| | | | | CN | 102215956 | A | |
| JP | 2000-501371 | A | 08 February 2000 | US | 6121187 | A | |
| | | | | entire text, all drawings | | | |
| | | | | WO | 1997/020630 | A1 | |
| | | | | EP | 876215 | A1 | |
| CN | 112316975 | A | 05 February 2021 | (Family: none) | | | |

Form PCT/ISA/210 (patent family annex) (January 2015)

**REFERENCES CITED IN THE DESCRIPTION**

**Patent documents cited in the description**

- JP 2002219362 A

- JP 2020218492 A